# NEW EUROPEAN PATENT SPECIFICATION

(11) **EP 0 693 687 B2**
(45) Date of publication and mention of the opposition decision: **04.06.2008**
(45) Mention of the grant of the patent: 28.07.1999
(21) Application number: 95114016.9
(22) Date of filing: 03.04.1991
(51) Int. Cl.: G01N 33/576, C07K 14/18

(54) **Combinations of hepatitis C virus (HCV) antigens for use in immunoassays for anti-HCV antibodies**
Kombinationen der Hepatitis-C-Virus (HCV) Antigene zur Verwendung in Immuntests für Anti-HCV-Antikörper
Combinaisons d'antigènes de virus hépatite-C (HCV) pour l'utilisation dans des essais immunologiques d'anticorps d'anti-HCV

(30) Priority: 04.04.1990 US 504352
(43) Date of publication of application: 24.01.1996
(62) Divisional of application: 91302910.4
(73) Proprietor: Novartis Vaccines and Diagnostics, Inc., Emeryville, CA 94608 (US)
(72) Inventor: Houghton, Michael, Danville, California 94526 (US); Choo, Qui-Lim, El Cerrito, California 94530 (US); Kuo, George, San Francisco, California 94112 (US)
(74) Representative: Voelker, Ingeborg Carla Emmy

(56) References cited:
- EP-A- 0 181 150
- EP-A- 0 318 216
- EP-A- 0 388 232
- EP-A- 0 445 423
- WO-A-89/04669

## Description

### Technical Field

The present invention is in the field of immunoassays for HCV (previously called Non-A, Non-B hepatitis virus). More particularly, it concerns combinations of HCV antigens that permit broad range immunoassays for anti-HCV antibodies.

### Background

The disease known previously as Non-A, Non-B hepatitis (NANBH) was considered to be a transmissible disease or family of diseases that were believed to be viral-induced, and that were distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH was due to a transmissible infectious agent or agents. Epidemiologic evidence suggested that there may be three types of NANBH: a water-borne epidemic type; a blood-borne or parenterally transmitted type; and a sporadically occurring (community acquired) type. However, until recently, no transmissible agent responsible for NANBH had been identified, and clinical diagnosis and identification of NANBH had been accomplished primarily by exclusion of other viral markers. Among the methods used to detect putative NANBH antigens and antibodies were agar-gel diffusion, counterimmunoelectrophoresis, immunofluorescence microscopy, immune electron microscopy, radioimmunoassay, and enzyme-linked immunosorbent assay. However, none of these assays proved to be sufficiently sensitive, specific, and reproducible to be used as a diagnostic test for NANBH.

In 1987, scientists at Chiron Corporation (the owner of the present application) identified the first nucleic acid definitively linked to blood-borne NANBH. See, e.g., EPO Pub. No. 318,216; Houghton et al., Science 244: 359 (1989). These publications describe therein the cloning of an isolate from a new viral class being named hepatitis C virus (HCV) the prototype virus described therein being named "HCV1." HCV is a flavi-like virus, with an RNA genome. EP-A-318,216 also describes the immunogenicity of the C100 polypeptide expressed from HCV cDNA and the use of HCV antigens in diagnostic immunoassays.

U.S. Patent No. 5,350,671 (Houghton et al.), describes the preparation of various recombinant HCV polypeptides by expressing HCV cDNA and the screening of those polypeptides for immunological reactivity with sera from HCV patients. That limited screening showed that at least five of the polypeptides tested were very immunogenic; specifically, those identified as 5-1-1, C100, C33c, CA279c, and CA290a. Of these five polypeptides, 5-1-1 is located in the putative NS4 domain; C100 spans the putative NS3 and NS4 domains; C33c is located within the putative NS3 domain and CA279a and CA290a are located within the putative C domain. The screening also showed that no single polypeptide tested was immunologically reactive with all sera. Thus, improved tests, which react with all or more samples from HCV positive individuals are desirable. WO 91/15574, published 17th October 1991, describes purified proteins and glycoproteins of HCV useful in a diagnostic system for detection of human HCV antisera.

### Disclosure of the Invention

Applicants have carried out additional serological studies on HCV antigens that confirm that no single HCV polypeptide identified to date is immunologically reactive with all sera. This lack of a single polypeptide that is universally reactive with all sera from individuals with HCV may be due, inter alia, to strain-to-strain variation in HCV epitopes, variability in the humoral response from individual-to-individual and/or variation in serology with the state of the disease.

These additional studies have also enabled applicants to identify combinations of HCV antigens that provide more efficient detection of HCV antibodies than any single HCV polypeptide.

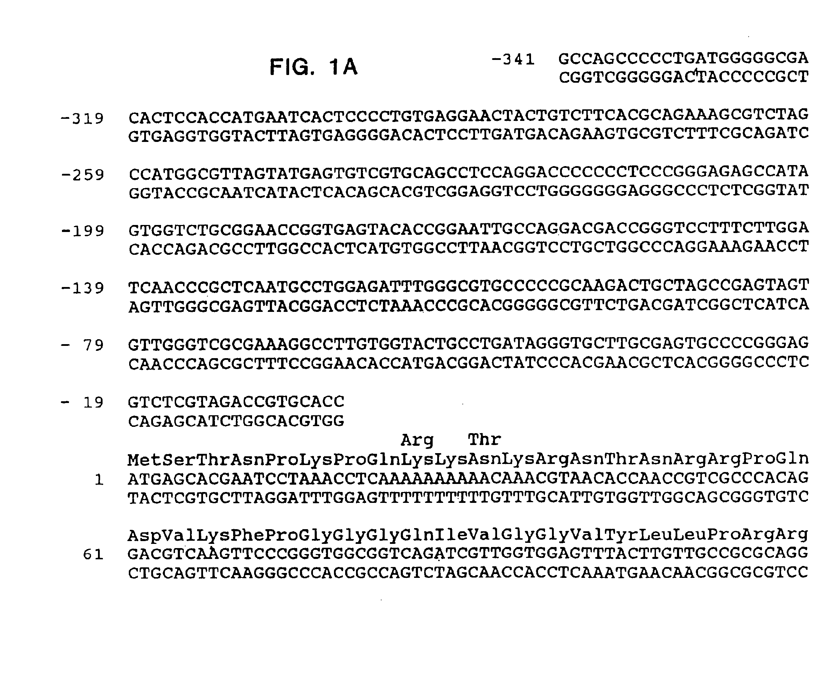

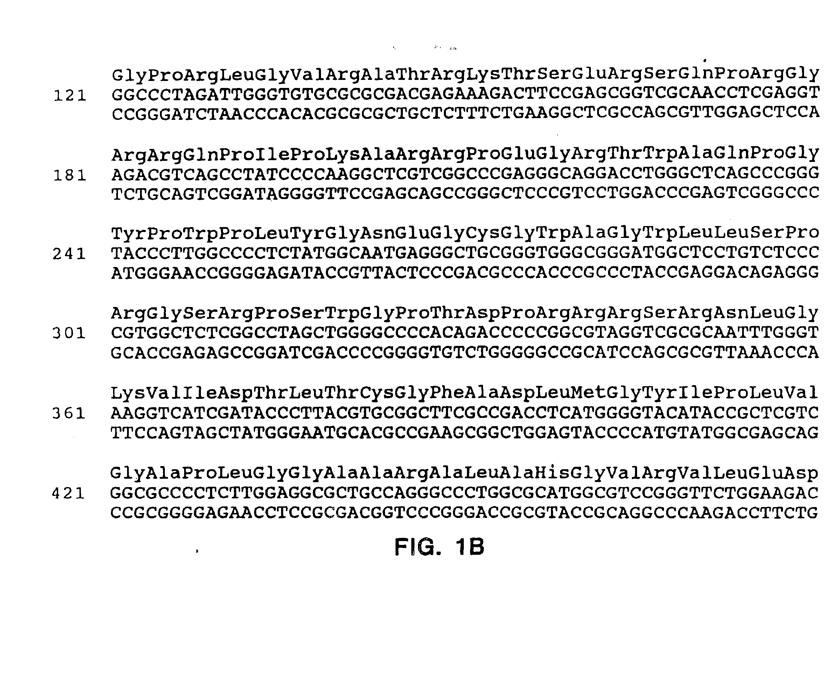

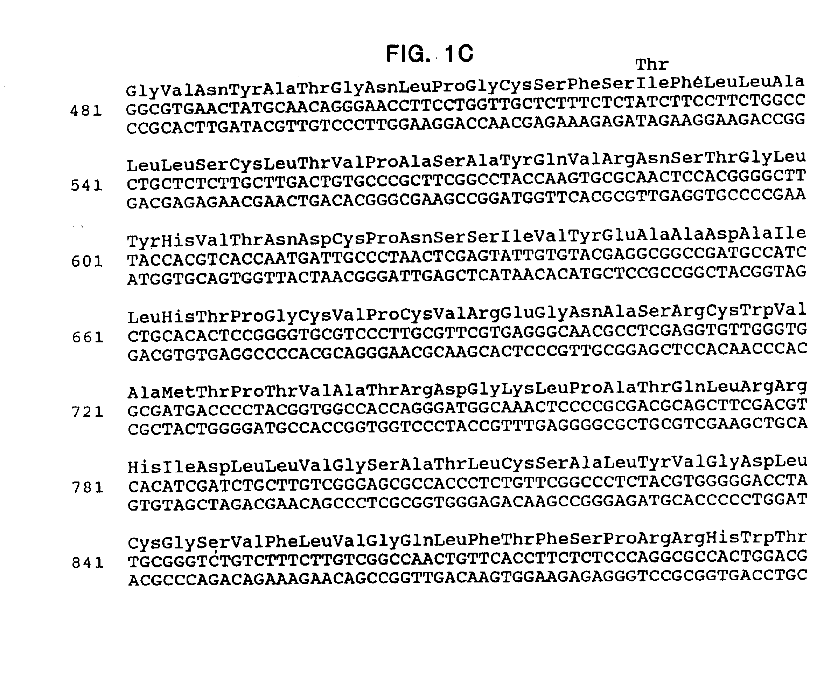

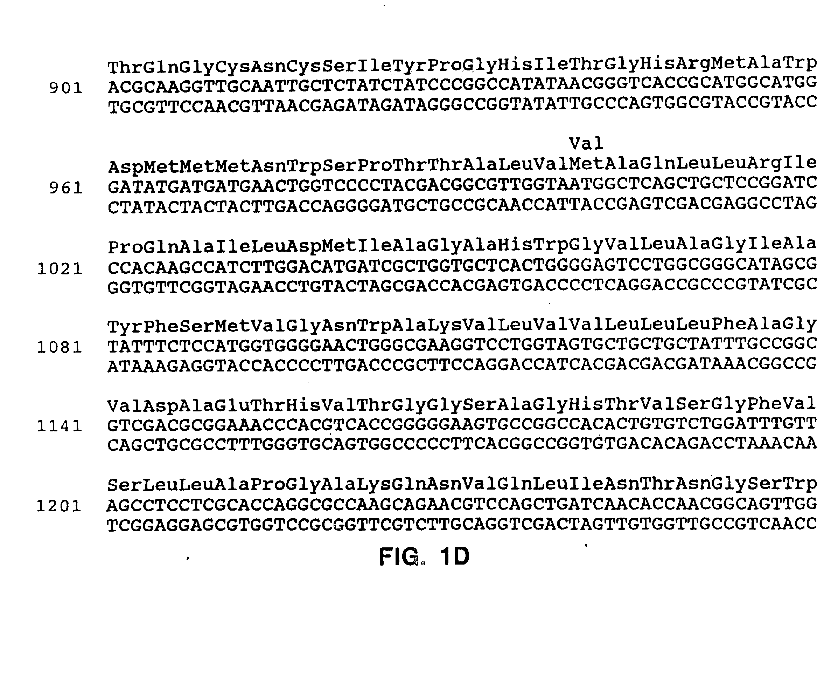

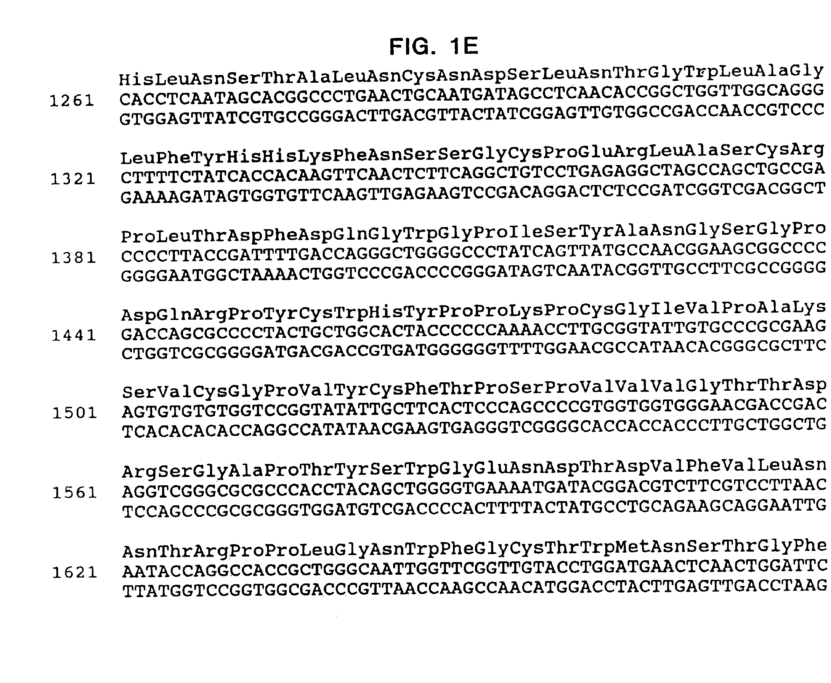

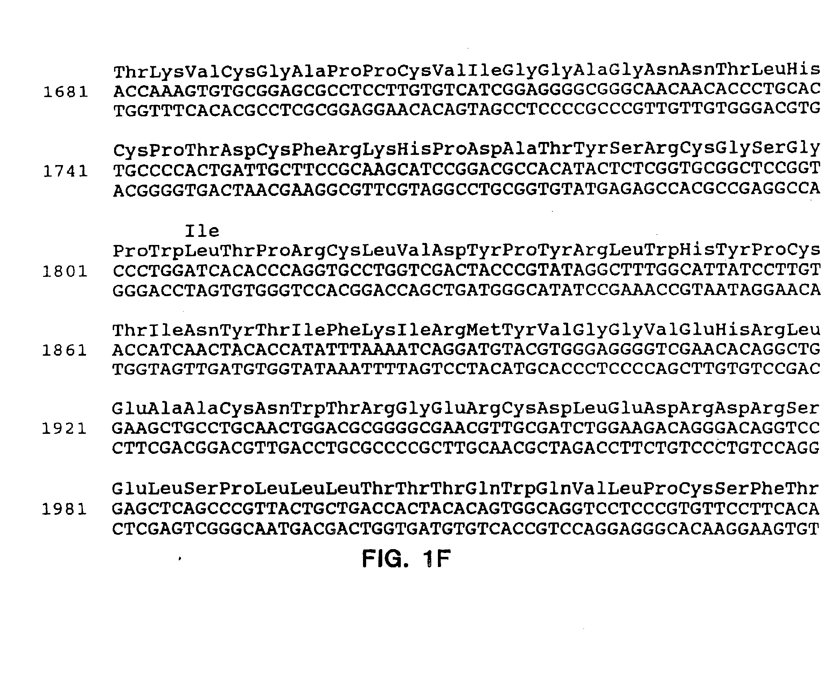

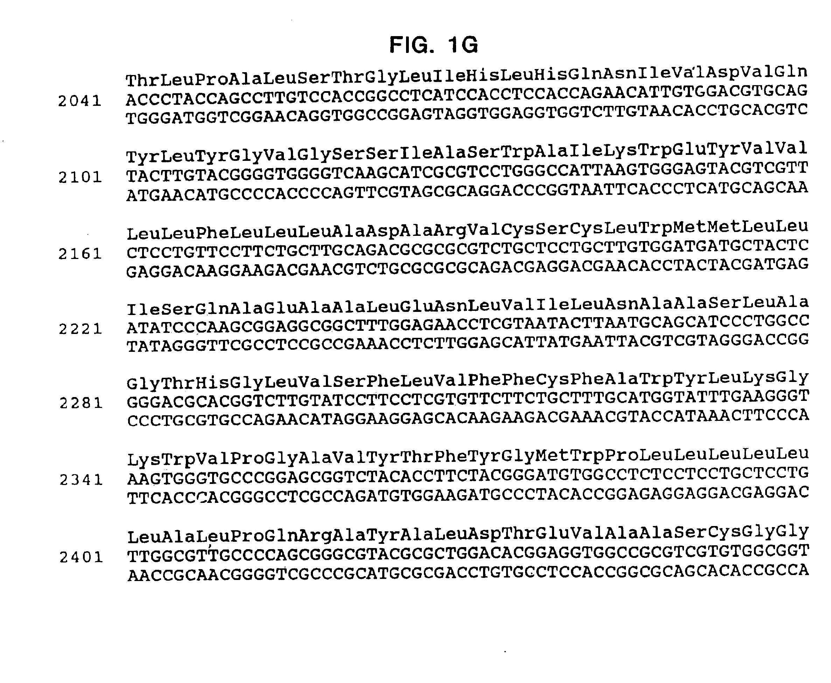

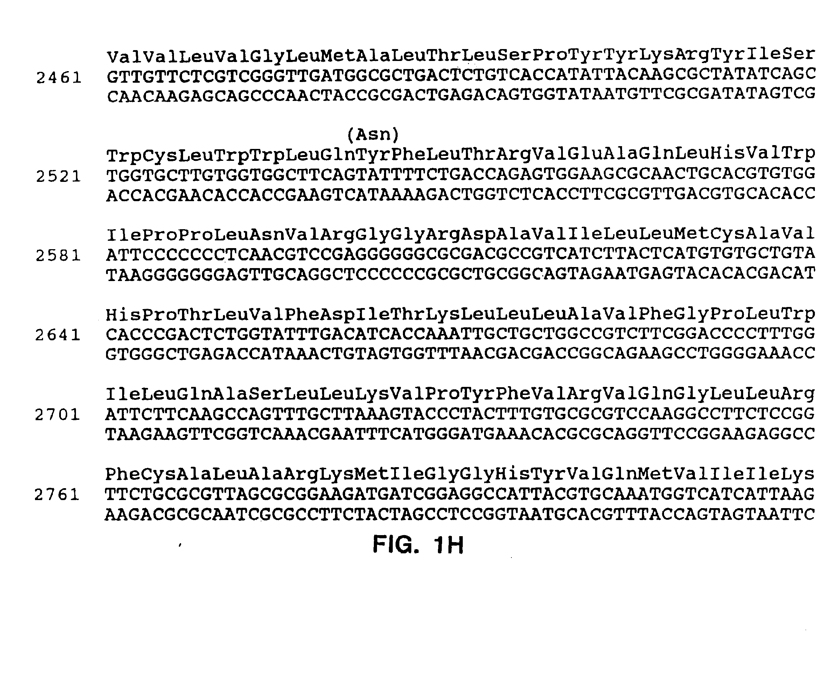

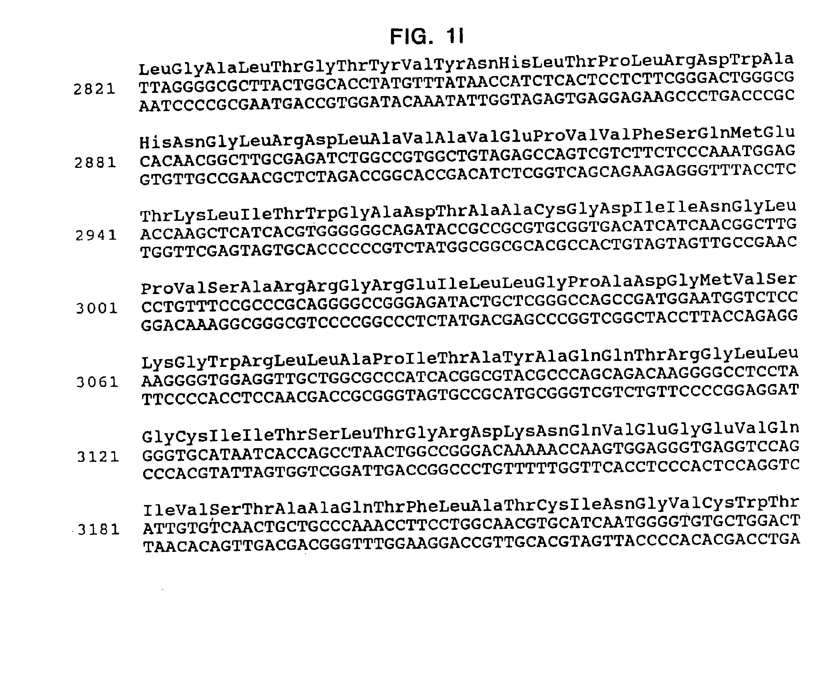

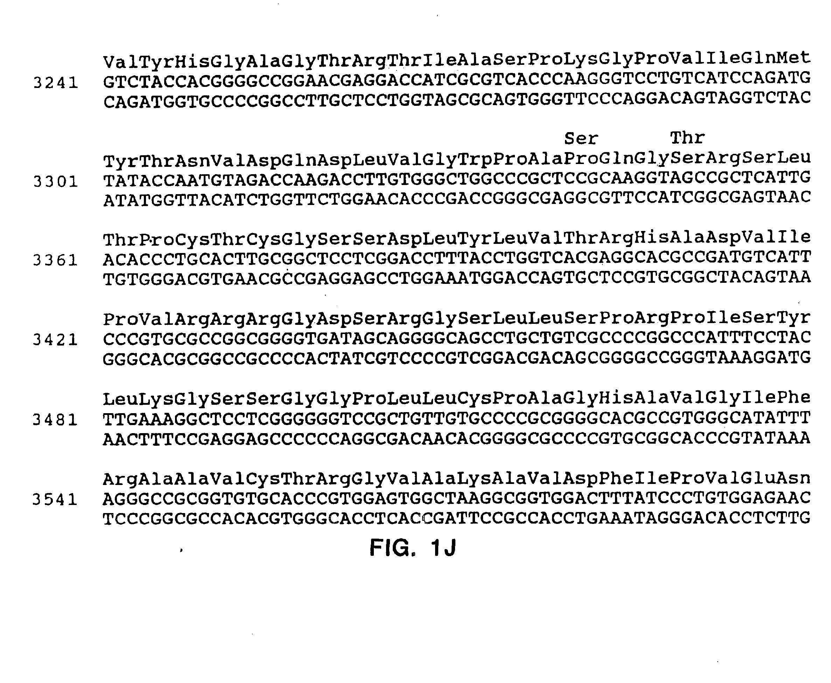

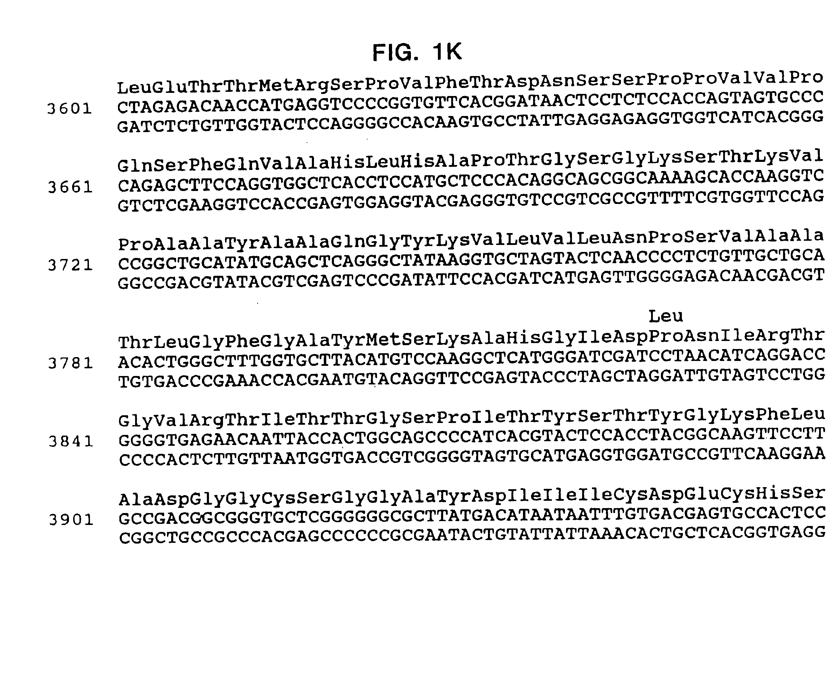

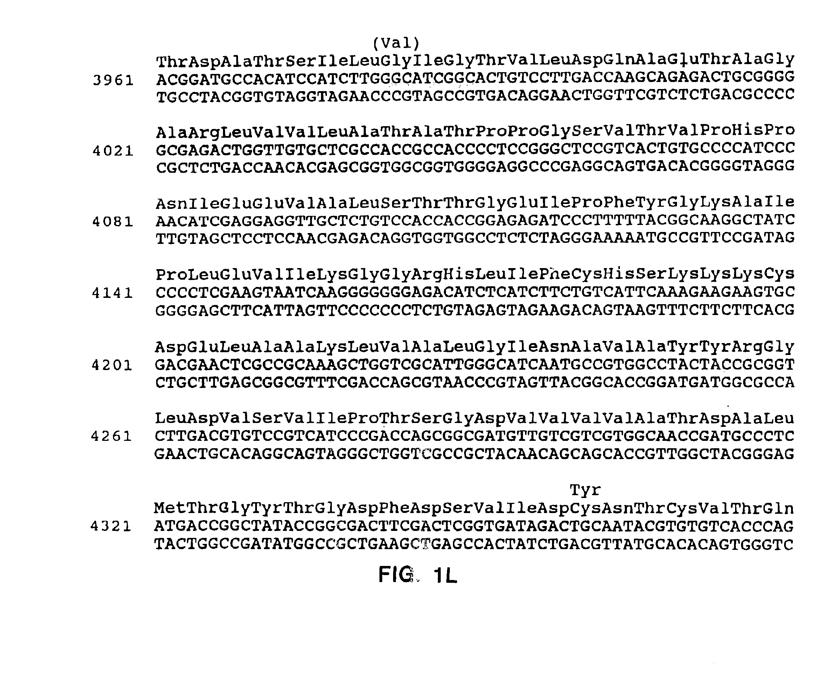

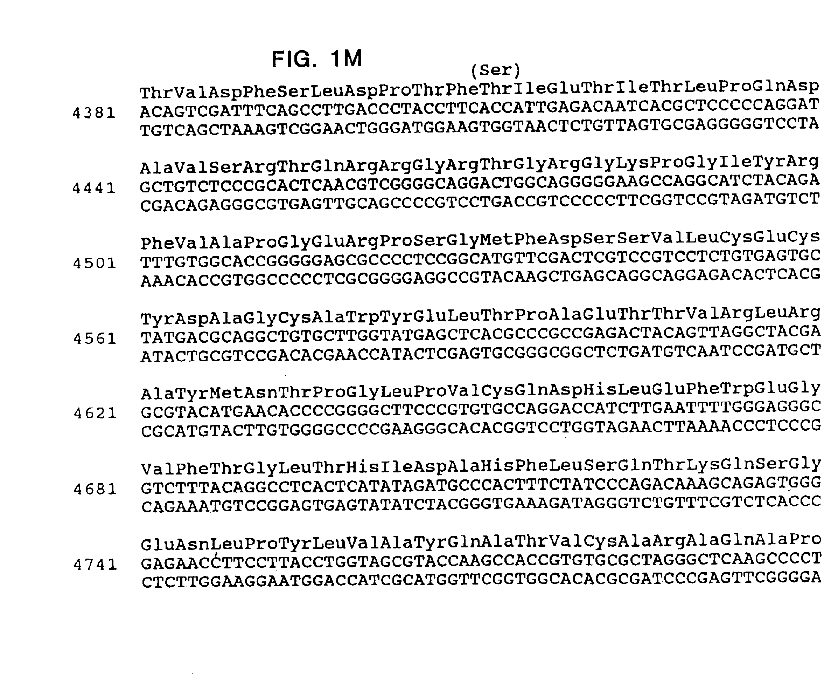

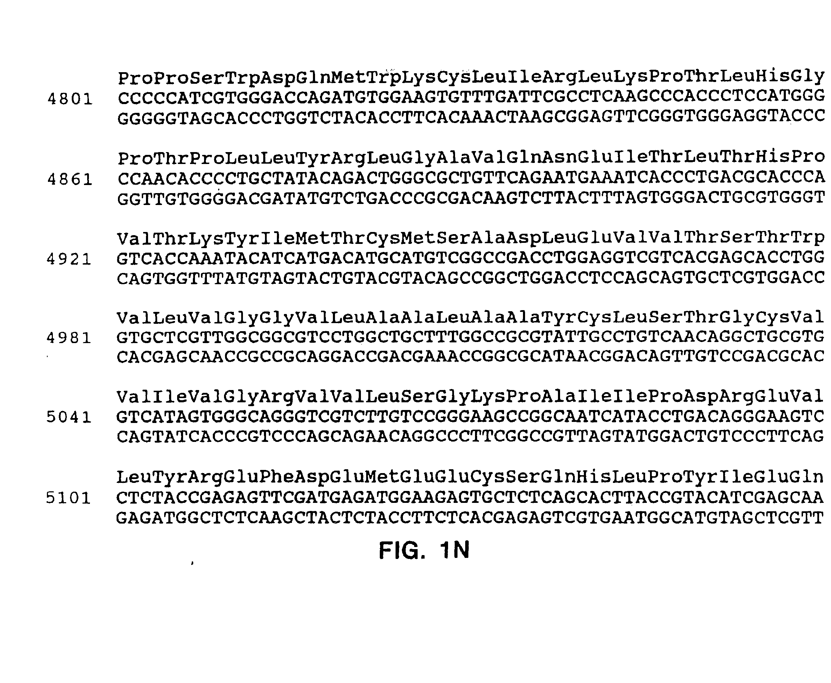

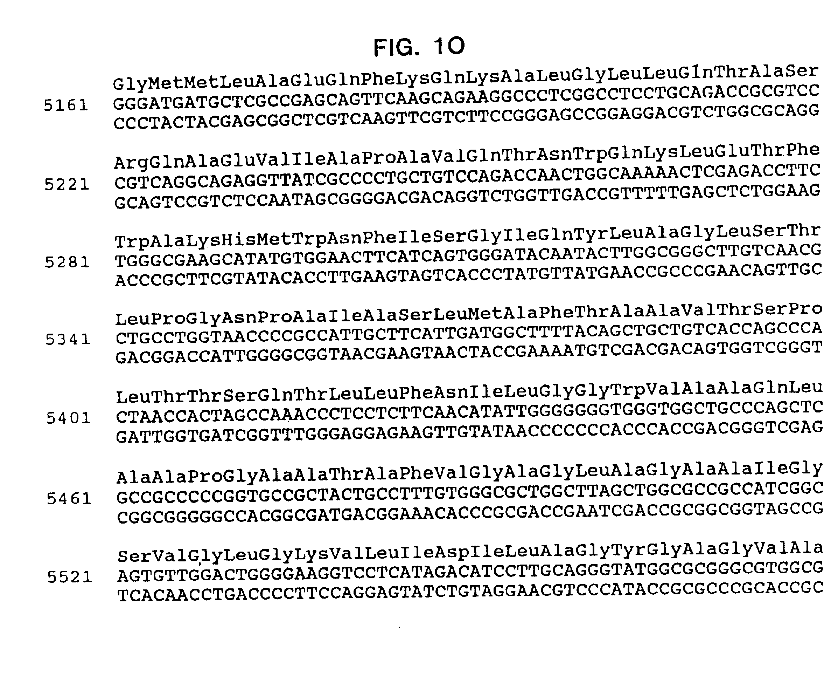

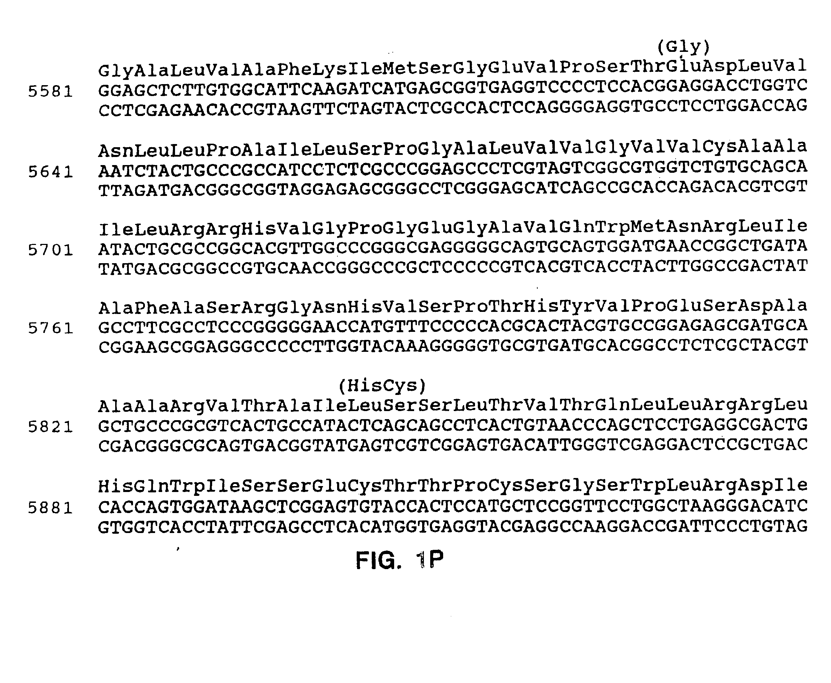

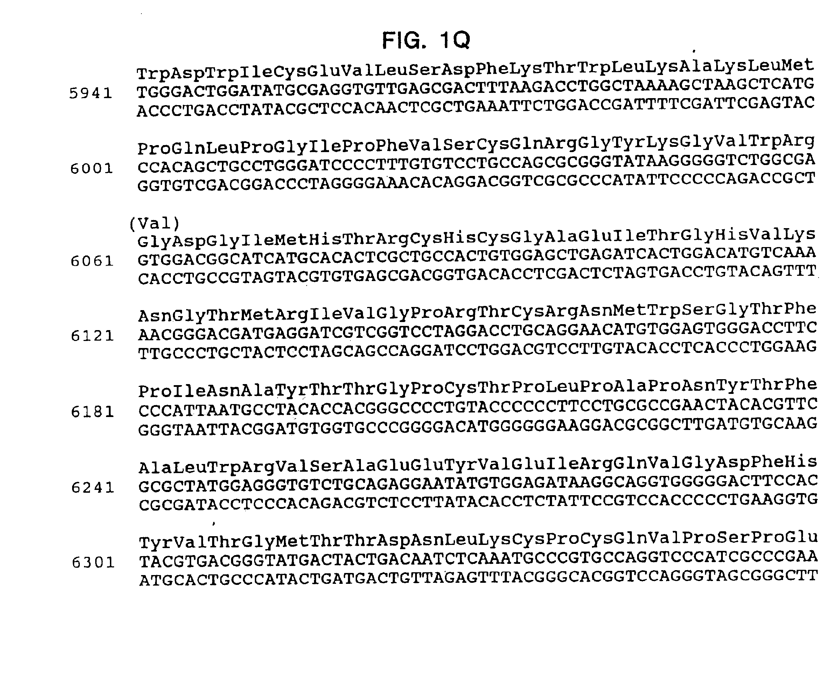

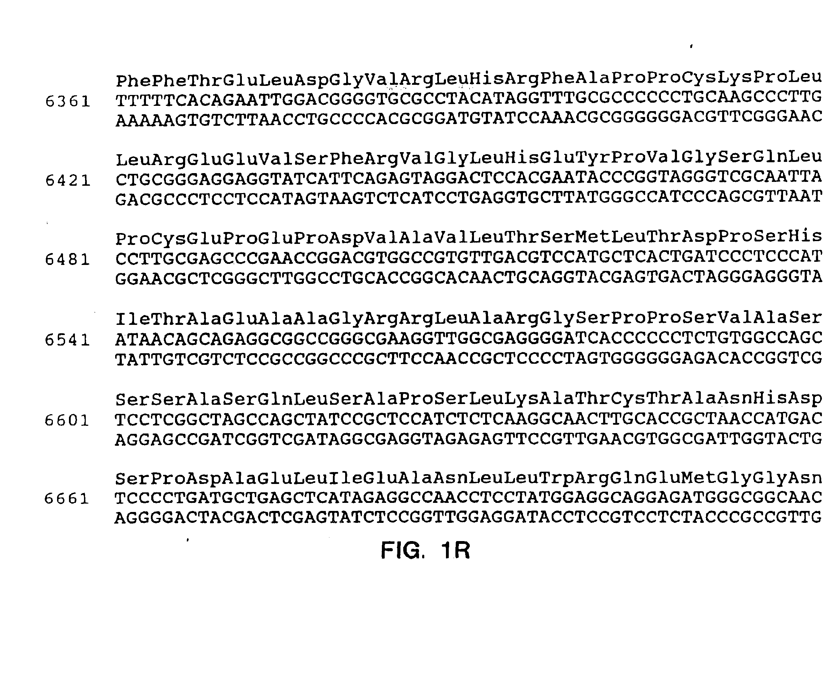

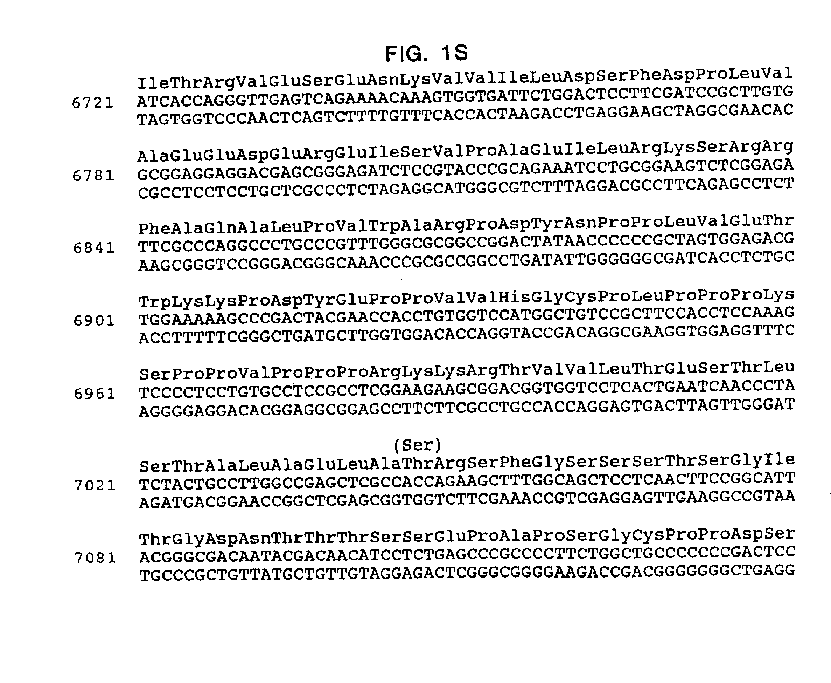

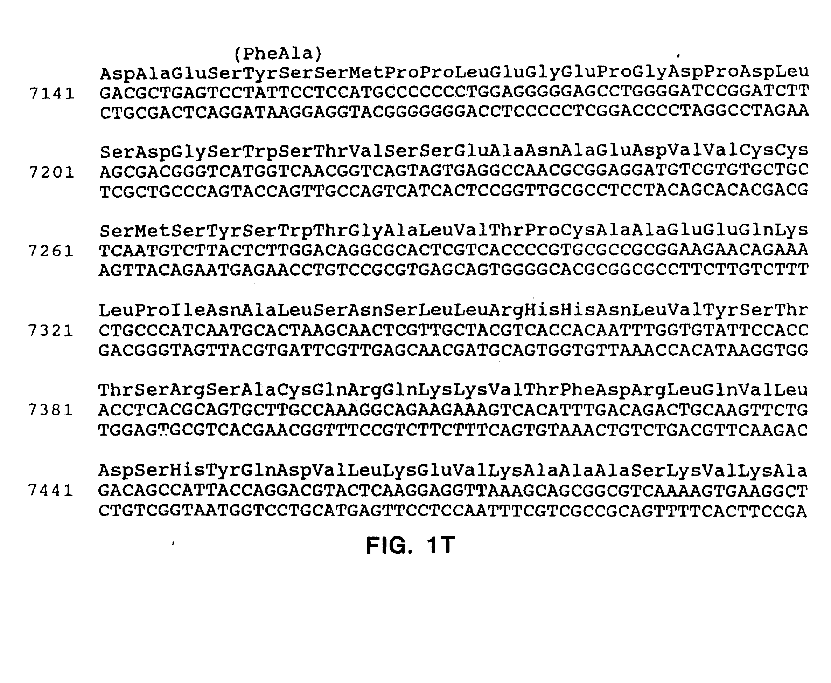

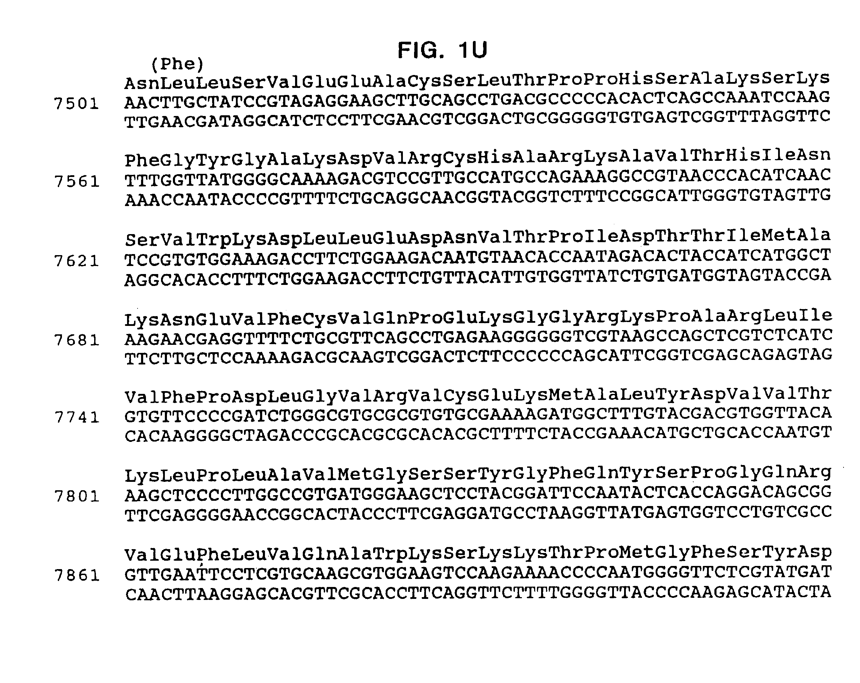

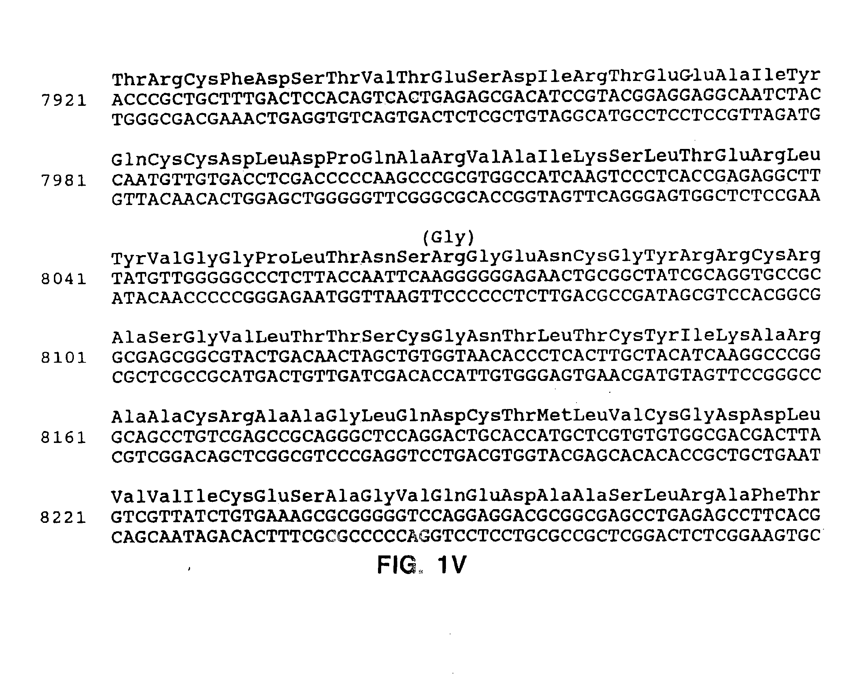

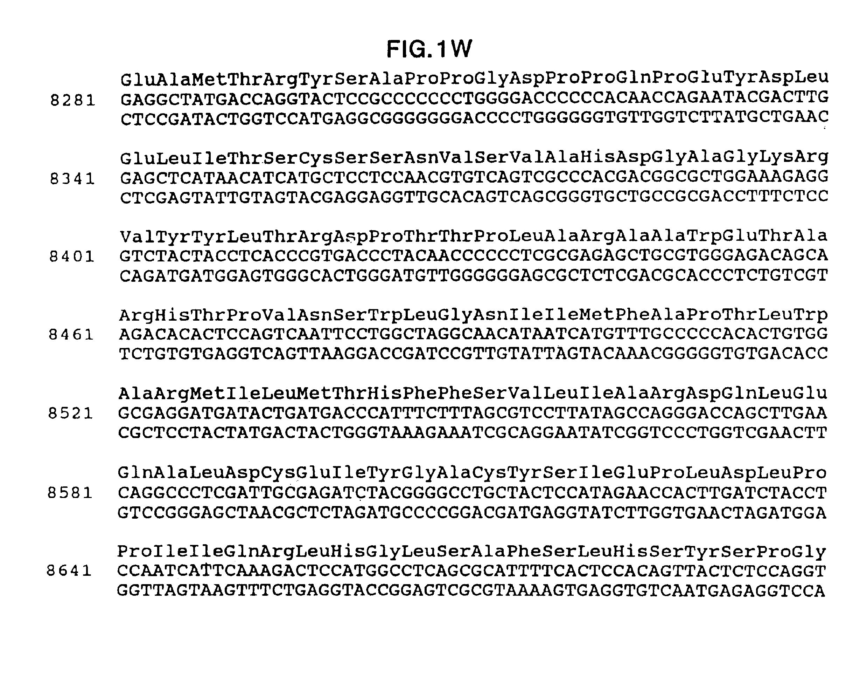

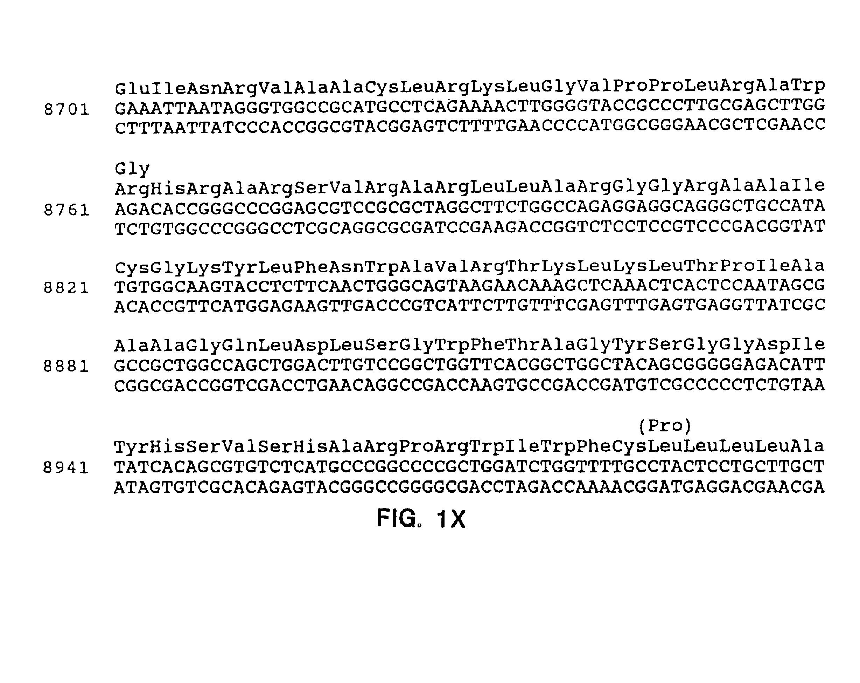

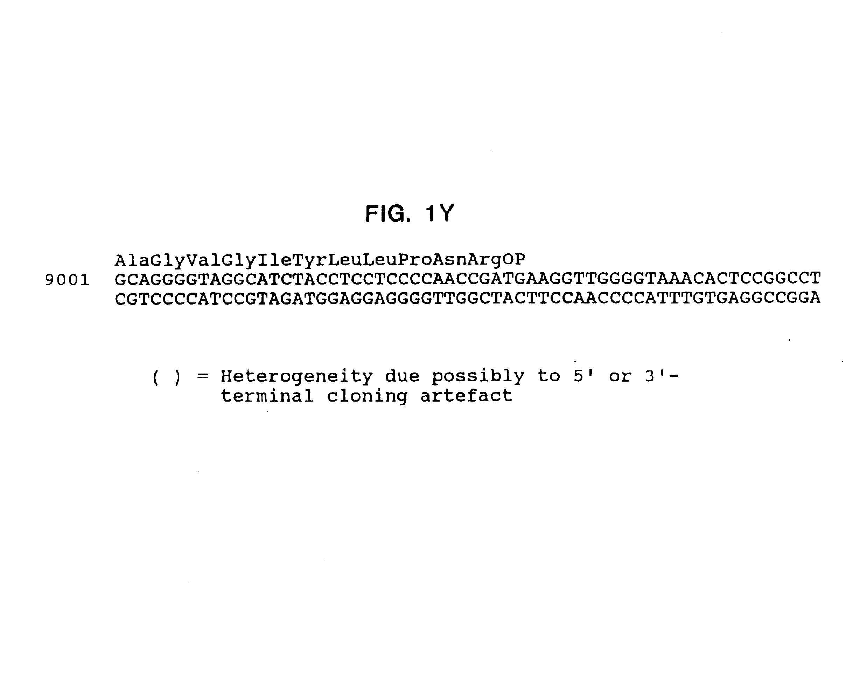

Accordingly, one aspect of this invention is a combination of hepatitis C virus (HCV) antigens in one or more polypeptides made by chemical synthesis or recombinant expression, comprising:
(a) a first HCV antigen comprising an epitope from the C domain of the HCV1 polyprotein shown in Figure 1; and
(b) a second HCV antigen selected from:
   (i) an HCV antigen comprising an epitope from the S domain of the HCV1 polyprotein shown in Figure 1;
   (ii) an HCV antigen comprising an epitope from the NS3 domain of the HCV1 polyprotein shown in Figure 1; (iii) an HCV antigen comprising an epitope from the NS4 domain of the HCV1 polyprotein shown in Figure 1; and
   (iv) an HCV antigen comprising an epitope from the NS5 domain of the HCV1 polyprotein shown in Figure 1;
   with the proviso that the combination is not the peptide p1 with C100-3, the peptide p35 with C100-3, the peptide p99 with C100-3 or a peptide having amino acids 9 to 177 of the HCV-1 polyprotein.

Preferably the second HCV antigen comprises an epitope from the S domain of the HCV1 polyprotein shown in Figure 1 and in this case, the combination preferably further comprises at least one additional HCV antigen selected from:
(i) an HCV antigen comprising an epitope from the NS3 domain of the HCV1 polyprotein shown in Figure 1;
(ii) an HCV antigen comprising an epitope from the NS4 domain of the HCV1 polyprotein shown in Figure 1;
(iii) an HCV antigen comprising an epitope from the NS5 domain of the HCV1 polyprotein shown in Figure 1;
More preferably, the additional antigen is from the NS3 domain of the HCV1 polyprotein shown in Figure 1.

Another aspect of the invention is a method for detecting antibodies to HCV in a mammalian body component suspected of containing said antibodies comprising contacting said body component with the above-described combination of HCV antigens under conditions that permit antibody-antigen reaction and detecting the presence of immune complexes of said antibodies and said antigens.

Another aspect of the invention is a method for detecting antibodies to HCV in a mammalian body component suspected of containing said antibodies comprising contacting said body component with a combination of HCV antigens of the invention under conditions that permit antibody-antigen reaction and detecting the presence of immune complexes of said antibodies and said antigens.

Another aspect of the invention is a kit for carrying out an assay for detecting antibodies to HCV in a mammalian body component suspected of containing said antibodies comprising in packaged combination.
(a) said combination of HCV antigens
(b) standard control reagents; and
(c) instructions for carrying out the assay.

### Brief Description of the Drawings

In the drawings:

Figure 1 is the nucleotide sequence of the cDNA sense and anti-sense strand for the HCV polyprotein and the amino acid sequence encoded by the sense strand.

Figure 2 is a schematic of the amino acid sequence of Figure 1 showing the putative domains of the HCV polypeptide.

### Modes for Carrying Out the Invention

### Definitions

"HCV antigen" means a polypeptide of at least about 5 amino acids, more usually at least about 8 to 10 amino acids that defines an epitope found in an isolate of HCV. Preferably, the epitope is unique to HCV. When an antigen is designated by an alphanumeric code, the epitope is from the HCV domain specified by the alphanumeric.

"Synthetic" as used to characterize an HCV antigen means that the HCV antigen has been man-made such as by chemical or recombinant synthesis.

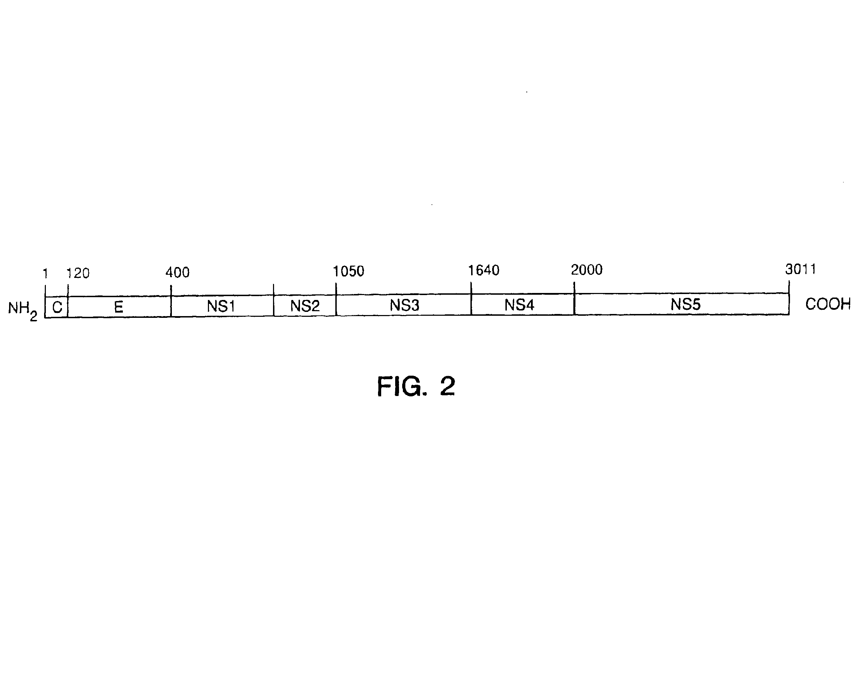

"Domains" means those segments of the HCV polyprotein shown in Figure 2 which generally correspond to the putative structural and nonstructural proteins of HCV. Domain designations generally follow the convention used to name Flaviviral proteins. The locations of the domains shown in Figure 2 are only approximate. The designations "NS" denotes "nonstructural" domains, while "S" denotes the envelope domain, and "C" denotes the nucleocapsid or core domain.

"Fusion polypeptide" means a polypeptide in which the HCV antigen(s) are part of a single continuous chain of amino acids, which chain does not occur in nature. The HCV antigens may be connected directly to each other by peptide bonds or be separated by intervening amino acid sequences. The fusion polypeptides may also contain amino acid sequences exogenous to HCV.

"Common solid matrix" means a solid body to which the individual HCV antigens or the fusion polypeptide comprised of HCV antigens are bound covalently or by noncovalent means such as hydrophobic adsorption.

"Mammalian body component" means a fluid or tissue of a mammalian individual (e.g., a human) that commonly contains antibodies produced by the individual. Such components are known in the art and include, without limitation, blood, plasma, serum, spinal fluid, lymph fluid, secretions of the respiratory, intestinal or genitourinary tracts, tears, saliva, milk, white blood cells, and myelomas.

"Immunologically reactive" means that the antigen in question will react specifically with anti-HCV antibody commonly present in a significant proportion of sera from individuals infected with HCV.

"Immune complex" means the combination or aggregate formed when an antibody binds to an epitope on an antigen.

### Combinations of HCV Antigens

Figure 2 shows the putative domains of the HCV polyprotein. The domains from which the antigens used in the combinations derive are: C, S (or E), NS3, NS4, and NS5. The C domain of the HCV1 polyprotein shown in Figure 1 is believed to define the nucleocapsid protein of HCV. It extends from the N-terminal of the polyprotein to approximately amino acid 120 of Figure 1. The S domain of the HCV1 polyprotein shown in Figure 1 is believed to define the virion envelope protein, and possibly the matrix (M) protein, and is believed to extend from approximately amino acid 120 to amino acid 400 of Figure 1. The NS3 domain of the HCV1 polyprotein shown in Figure 1 extends from approximately amino acid 1050 to amino acid 1640 and is believed to constitute the viral protease. The NS4 domain of the HCV1 polyprotein shown in Figure 1 extends from the terminus of NS3 to approximately amino acid 2000. The function of the NS4 protein is not known at this time. Finally, the NS5 domain of the HCV1 polyprotein shown in Figure 1 extends from about amino acid 2000 to the end of the polyprotein and is believed to define the viral polymerase.

The sequence shown in Figure 1 is the sequence of the HCV1 isolate. It is expected that the sequences of other strains of the blood-borne HCV may differ from the sequence of Figure 1, particularly in the envelope (S) and nucleocapsid (C) domains.

In general, the HCV antigens will comprise entire or truncated domains, the domain fragments being readily screened for antigenicity by those skilled in the art. The individual HCV antigens used in the combination will preferably comprise the immunodominant portion (i.e., the portion primarily responsible for the immunological reactivity of the polypeptide) of the stated domain. In the case of the C domain it is preferred that the C domain antigen comprise a majority of the entire sequence of the domain. The antigen designated C22 (see Example 4, infra), is particularly preferred. The S domain antigen preferably includes the hydrophobic subdomain at the N-terminal end of the domain. This hydrophobic subdomain extends from approximately amino acid 199 to amino acid 328 of Figure 1. The HCV antigen designated S2 (see Example 3, infra), is particularly preferred. Sequence downstream of the hydrophobic subdomain may be included in the S domain antigen if desired.

A preferred NS3 domain antigen is the antigen designated C33c. That antigen includes amino acids 1192 to 1457 of Figure 1. A preferred NS4 antigen is C100 which comprises amino acids 1569 to 1931 of Figure 1. A preferred NS5 antigen comprises amino acids 2054 to 2464 of Figure 1.

The HCV antigen may be in the form of a polypeptide composed entirely of HCV amino acid sequence or it may contain sequence exogenous to HCV (i.e., it may be in the form of a fusion protein that includes exogenous sequence). In the case of recombinantly produced HCV antigen, producing the antigen as a fusion protein such as with SOD, alpha-factor or ubiquitin (see commonly owned U.S. Pat. No. 4,751,180, U.S. Pat. No. 4,870,008 and U.S. Pat. Application Serial. No. 390,599, filed 7 August 1989, which describe expression of SOD, alpha-factor and ubiquitin fusion proteins) may increase the level of expression and/or increase the water solubility of the antigen. Fusion proteins such as the alpha-factor and ubiquitin fusion are processed by the expression host to remove the heterologous sequence. Alpha-factor is a secretion system, however, while ubiquitin fusions remain in the cytoplasm.

Further, the combination of antigens may be produced as a fusion protein. For instance, a continuous fragment of DNA encoding C22 and C33c may be constructed, cloned into an expression vector and used to express a fusion protein of C22 and C33c. In a similar manner fusion proteins of C22 and C100; C22 and S2; C22 and an NS5 antigen; C22, C33c, and S2; C22, C100 and S2, and C22, C33c, C100, and S2 may be made. Alternative fragments from the exemplified domain may also be used.

### Preparation of HCV Antigens

The HCV antigens of the invention are produced recombinantly or by known solid phase chemical synthesis.

When produced by recombinant techniques, standard procedures for constructing DNA encoding the antigen, cloning that DNA into expression vectors, transforming host cells such as bacteria, yeast, insect, or mammalian cells, and expressing such DNA to produce the antigen may be employed. As indicated previously, it may be desirable to express the antigen as a fusion protein to enhance expression, facilitate purification, or enhance solubility. Examples of specific procedures for producing representative HCV antigens are described in the Examples, infra, and in US Patent 5,350,671.

### Formulation of Antigens for Use in Immunoassay

The HCV antigens may be combined by producing them in the form of a fusion protein composed of two or more of the antigens, by immobilizing them individually on a common solid matrix, or by physically mixing them. Fusion proteins of the antigen may also be immobilized on (bound to) a solid matrix. Methods and means for covalently or noncovalently binding proteins to solid matrices are known in the art. The nature of the solid surface will vary depending upon the assay format. For assays carried out in microtiter wells, the solid surface will be the wall of the well or cup. For assays using beads, the solid surface will be the surface of the bead. In assays using a dipstick (i.e., a solid body made from a porous or fibrous material such as fabric or paper) the surface will be the surface of the material from which the dipstick is made. In agglutination assays the solid surface may be the surface of latex or gelatin particles. When individual antigens are bound to the matrix they may be distributed homogeneously on the surface or distributed thereon in a pattern, such as bands so that a pattern of antigen binding may be discerned.

Simple mixtures of the antigens comprise the antigens in any suitable solvent or dispersing medium.

### Assay Formats Using Combinations of Antigens

The HCV antigens may be employed in virtually any assay format that employs a known antigen to detect antibodies. A common feature of all of these assays is that the antigen is contacted with the body component suspected of containing HCV antibodies under conditions that permit the antigen to bind to any such antibody present in the component. Such conditions will typically be physiologic temperature, pH and ionic strength using an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labeled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin, and enzyme-labeled and mediated immunoassays, such as ELISA assays.

The immunoassay may be, without limitation, in a heterogenous or in a homogeneous format, and of a standard or competitive type. In a heterogeneous format, the polypeptide is typically bound to a solid matrix or support to facilitate separation of the sample from the polypeptide after incubation. Examples of solid supports that can be used are nitrocellulose (e.g., in membrane or microtiter well form), polyvinyl chloride (e.g., in sheets or microtiter wells), polystyrene latex (e.g., in beads or microtiter plates, polyvinylidine fluoride (known as Immulon™), diazotized paper, nylon membranes, activated beads, and Protein A beads. For example, Dynatech Immulon™ 1 or Immulon™ 2 microtiter plates or 6.4 mm (0.25 inch) polysterene beads (Precision Plastic Ball) can be used in the heterogeneous format. The solid support containing the antigenic polypeptides is typically washed after separating it from the test sample, and prior to detection of bound antibodies. Both standard and competitive formats are known in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of HCV antibodies forming the antibody-antigen complex is directly monitored. This may be accomplished by determining whether labeled anti-xenogenic (e.g., anti-human) antibodies which recognize an epitope on anti-HCV antibodies will bind due to complex formation. In a competitive format, the amount of HCV antibodies in the sample is deduced by monitoring the competitive effect on the binding of a known amount of labeled antibody (or other competing ligand) in the complex.

Complexes formed comprising anti-HCV antibody (or, in the case of competetive assays, the amount of competing antibody) are detected by any of a number of known techniques, depending on the format. For example, unlabeled HCV antibodies in the complex may be detected using a conjugate of antixenogeneic Ig complexed with a label, (e.g., an enzyme label).

In an immunoprecipitation or agglutination assay format the reaction between the HCV antigens and the antibody forms a network that precipitates from the solution or suspension and forms a visible layer or film of precipitate. If no anti-HCV antibody is present in the test specimen, no visible precipitate is formed.

The HCV antigens will typically be packaged in the form of a kit for use in these immunoassays. The kit will normally contain in separate containers the combination of antigens (either already bound to a solid matrix or separate with reagents for binding them to the matrix), control antibody formulations (positive and/or negative), labeled antibody when the assay format requires same and signal generating reagents (e.g., enzyme substrate) if the label does not generate a signal directly. Instructions (e.g., written, tape, VCR, CD-ROM, etc.) for carrying out the assay usually will be included in the kit.

The following examples are intended to illustrate the invention and are not intended to limit the invention in any manner.

### Example 1: Synthesis of HCV Antigen C33c

HCV antigen C33c contains a sequence from the NS3 domain. Specifically, it includes amino acids 1192-1457 of Figure 1. This antigen was produced in bacteria as a fusion protein with human superoxide dismutase (SOD) as follows. The vector pSODcf1 (Steiner et al. (1986), J. Virol. 58:9) was digested to completion with EcoRI and BamHI and the resulting EcoRI,BamHI fragment was ligated to the following linker to form pcf1EF: 
GATC CTG GAA TTC TGA TAA
GAC CTT AAG ACT ATT TTA A
 A cDNA clone encoding amino acids 1192-1457 and having EcoRI ends was inserted into pcf1EF to form pcf1EF/C33c. This expression construct was transformed into D1210 E. coli cells.

The transformants were used to express a fusion protein comprised of SOD at the N-terminus and in-frame C33c HCV antigen at the C-terminus. Expression was accomplished by inoculating 1500 ml of Luria broth containing ampicillin (100 micrograms/ml) with 15 ml of an overnight culture of the transformants. The cells were grown to an O.D. of 0.3, IPTG was added to yield a final concentration of 2 mM, and growth continued until the cells attained a density of 1 O.D., at which time they were harvested by centrifugation at 3,000 x g at 4°C for 20 minutes. The packed cells can be stored at -80°C for several months.

In order to purify the SOD-C33c polypeptide the bacterial cells in which the polypeptide was expressed were subjected to osmotic shock and mechanical disruption, the insoluble fraction containing SOD-C33c was isolated and subjected to differential extraction with an alkaline-NaCl solution, and the fusion polypeptide in the extract purified by chromatography on columns of S-Sepharose^{(TM)} and Q-Sepharose^{(TM)}.

The crude extract resulting from osmotic shock and mechanical disruption was prepared by the following procedure. One gram of the packed cells were suspended in 10 ml of a solution containing 0.02 M Tris HCl, pH 7.5, 10 mM EDTA, 20% sucrose, and incubated for 10 minutes on ice. The cells were then pelleted by centrifugation at 4,000 x g for 15 min at 4°C. After the supernatant was removed, the cell pellets were resuspended in 10 ml of Buffer A1 (0.01M Tris HCl, pH 7.5, 1 mM EDTA, 14 mM betamercaptoethanol [BME]), and incubated on ice for 10 minutes. The cells were again pelleted at 4,000 x g for 15 minutes at 4°C. After removal of the clear supernatant (periplasmic fraction I), the cell pellets were resuspended in Buffer A1, incubated on ice for 10 minutes, and again centrifuged at 4,000 x g for 15 minutes at 4°C. The clear supernatant (periplasmic fraction II) was removed, and the cell pellet resuspended in 5 ml of Buffer A2 (0.02 M Tris HCl, pH 7.5, 14 mM BME, 1 mM EDTA, 1 mM PMSF). In order to disrupt the cells, the suspension (5 ml) and 7.5 ml of Dyno-mill™ lead-free acid washed glass beads (0.10-0.15 mm diameter)(obtained from Glen-Mills, Inc.) were placed in a Falcon™ tube, and vortexed at top speed for two minutes, followed by cooling for at least 2 min on ice; the vortexing-cooling procedure was repeated another four times. After vortexing, the slurry was filtered through a scintered glass funnel using low suction; the glass beads were washed two times with Buffer A2, and the filtrate and washes combined.

The insoluble fraction of the crude extract was collected by centrifugation at 20,000 x g for 15 min at 4°C, washed twice with 10 ml Buffer A2, and resuspended in 5 ml of MILLI-Q^{(TM)} water.

A fraction containing SOD-C33c was isolated from the insoluble material by adding to the suspension NaOH (2 M) and NaCl (2 M) to yield a final concentration of 20 mM each, vortexing the mixture for 1 minute, centrifuging it 20,000 x g for 20 min at 4°C, and retaining the supernatant.

In order to purify SOD-C33c on S-Sepharose^{(TM)}, the supernatant fraction was adjusted to a final concentration of 6M urea, 0.05M Tris HCl, pH 7.5, 14 mM BME, 1 mM EDTA. This fraction was then applied to a column of S-Sepharose^{(TM)} Fast Flow (1.5 x 10 cm) which had been equilibrated with Buffer B (0.05M Tris HCl, pH 7.5, 14 mM BME, 1 mM EDTA). After application, the column was washed with two column volumes of Buffer B. The flow through and wash fractions were collected. The flow rate of application and wash, was 1 ml/min; and collected fractions were 1 ml. In order to identify fractions containing SOD-C33c, aliquots of the fractions were analyzed by electrophoresis on 10% polyacrylamide gels containing SDS followed by staining with Coomassie blue. The fractions are also analyzable by Western blots using an antibody directed against SOD. Fractions containing SOD-C33c were pooled.

Further purification of SOD-C33c was on a Q-Sepharose™ column (1.5 x 5 cm) which was equilibrated with Buffer B. The pooled fractions containing SOD-C33c obtained from chromatography on S-Sepharose was applied to the column. The column was then washed with Buffer B, and eluted with 60 ml of a gradient of 0.0 to 0.4 M NaCl in Buffer B. The flow rate for application, wash, and elution was 1 ml/min; collected fractions were 1 ml. All fractions from the Q-Sepharose™ column were analyzed as described for the S-Sepharose™ column. The peak of SOD-C33c eluted from the column at about 0.2 M NaCl.

The SOD-C33c obtained from the Q-Sepharose™ column was greater than about 90% pure, as judged by analysis on the polyacrylamide SDS gels and immunoblot using a monoclonal antibody directed against human SOD.

### Example 2: Synthesis of HCV Antigen C100

HCV antigen C100 contains sequences from the NS3 and NS4 domains. Specifically, it includes amino acids 1569-1931 of Figure 1. This antigen was produced in yeast. A cDNA fragment of a 1270 bp encoding the above amino acids and heaving EcoRI termini was prepared.

The construction of a yeast expression vector in which this fragment was fused directly to the S. cerevisiae ADH2/GAP promoter was accomplished by a protocol which included amplification of the C100 sequence using a PCR method, followed by ligation of the amplified sequence into a cloning vector. After cloning, the C100 sequence was excised, and with a sequence which contained the ADH2/GAP promoter, was ligated to a large fragment of a yeast vector to yield a yeast expression vector.

The PCR amplification of C100 was performed using as template the vector pS3-56_{C100m}, which had been linearized by digestion with SalI. pS3-56, which is a pBR322 derivative, contains an expression cassette which is comprised of the ADH2/GAPDH hybrid yeast promoter upstream of the human superoxide dismutase gene, and a downstream alpha factor transcription terminator.

The oligonucleotide primers used for the amplification were designed to facilitate cloning into the expression vector, and to introduce a translation termination codon. Specifically, novel 5'-HindIII and 3'-SalI sites were generated with the PCR oligonucleotides. The oligonucleotide containing the SalI site also encodes the double termination codons, TAA and TGA. The oligonucleotide containing the HindIII site also contains an untranslated leader sequence derived from the pgap63 gene, situated immediately upstream of the AUG codon. The pEco63GAPDH gene is described by Holland and Holland (1980) and by Kniskern et al. (1986). The PCR primer sequences used for the direct expression of C100m were: 
5' GAG TGC TCA AGC TTC AAA ACA AAA TGG CTC
ACT TTC TAT CCC AGA CAA AGC AGA GT 3'
 and

5′ GAG TGC TCG TCG ACT CAT TAG GGG GAA
ACA TGG TTC CCC CGG GAG GCG AA 3′.

Amplification by PCR, utilizing the primers, and template, was with a Cetus-Perkin-Elmer PCR kit, and was performed according to the manufacturer's directions. The PCR conditions were 29 cycles of 94°C for a minute, 37°C for 2 minutes, 72°C for 3 minutes; and the final incubation was at 72°C for 10 minutes. The DNA can be stored at 4°C or -20°C overnight.

After amplification, the PCR products were digested with HindIII and SalI. The major product of 1.1 kb was purified by electrophoresis on a gel, and the eluted purified product was ligated with a large SalI-HindIII fragment of pBR322. In order to isolate correct recombinants, competent HB101 cells were transformed with the recombinant vectors, and after cloning, the desired recombinants were identified on the basis of the predicted size of HindIII- SalI fragments excised from the clones. One of the clones which contained the a HindIII-SalI fragment of the correct size was named pBR322/C100⁻d. Confirmation that this clone contained amplified C100 was by direct sequence analysis of the HindIII-SalI fragment.

The expression vector containing C100 was constructed by ligating the HindIII-SalI fragment from pBR322/C100⁻ d to a 13.1 kb BamHI-SalI fragment of pBS24.1, and a 1369 bm BamHI-HindIII fragment containing the ADH2/GAP promoter. (The latter fragment is described in EPO 164,556). The ADH2/GAP promoter fragment was obtained by digestion of the vector pPGAP/AG/HindIII with HindIII and BamHI, followed by purification of the 1369 bp fragment on a gel.

Competent HB101 cells were transformed with the recombinant vectors; and correct recombinants were identified by the generation of a 2464 bp fragment and a 13.1 kb fragment generated by BamHI and SalI digestion of the cloned vectors. One of the cloned correct recombinant vectors was named pC100⁻d#3.

In order to express C100, competent cells of Saccharomyces cerevisiae strain AB122 (MATa leu2 ura3-53 prb 1-1122 pep4-3 prcl-407[cir-0]) were transformed with the expression vector pC100⁻d#3. The transformed cells were plated on URA-sorbitol, and individual transformants were then streaked on Leu⁻ plates.

Individual clones were cultured in Leu⁻, ura⁻ medium with 2% glucose at 30°C for 24-36 hours. One liter of Yeast Extract Peptone Medium (YEP) containing 2% glucose was inoculated with 10 ml of the overnight culture, and the resulting culture was grown at 30°C at an agitation rate of 400 rpm and an aeration rate of 1 L of air per 1 L of medium per minute (i.e., 1vvm) for 48 hours. The pH of the medium was not controlled. The culture was grown in a BioFlo II fermentor manufactured by New Brunswick Science Corp. Following fermentation, the cells were isolated and analyzed for C100 expression.

Analysis for expressed C100 polypeptide by the transformed cells was performed on total cell lysates and crude extracts prepared from single yeast colonies obtained from the Leu⁻ plates. The cell lysates and crude extracts were analyzed by electrophoresis on SDS polyacrylamide gels, and by Western blots. The Western blots were probed with rabbit polyclonal antibodies directed against the SOD-C100 polypeptide expressed in yeast. The expected size of the C100 polypeptide is 364 amino acids. By gel analysis the expressed polypeptide has a MWᵣ of 39.9K.

Both analytical methods demonstrated that the expressed C100 polypeptide was present in total cell lysates, but was absent from crude extracts. These results suggest that the expressed C100 polypeptide may be insoluble.

### Example 3: Expression of HCV Antigen S2

HCV antigen S2 contains a sequence from the hydrophobic N-terminus of the S domain. It includes amino acids 199-328 of Figure 1.

The protocol for the construction of the expression vector encoding the S2 polypeptide and for its expression in yeast was analogous to that used for the expression of the C100 polypeptide, described in Example 2.

The template for the PCR reaction was the vector pBR322/Pi14a, which had been linearized by digestion with HindIII. Pi14a is a cDNA clone that encodes amino acids 199-328.

The oligonucleotides used as primers for the amplification by PCR of the S2 encoding sequence were the following.
For the 5'-region of the S2 sequence: 
5' GAG TGC TCA AGC TTC AAA ACA AAA TGG GGC TCT
ACC ACG TCA CCA ATG ATT GCC CTA AC 3';
 and
for the 3'-region of the S2 sequence: 
5'GAG TGC TCG TCG ACT CAT TAA GGG GAC CAG TTC
ATC ATC ATA TCC CAT GCC AT 3'.
 The primer for the 5'-region introduces a HindIII site and an ATG start codon into the amplified product. The primer for the 3'-region introduces translation stop codons and a SalI site into the amplified product.

The PCR conditions were 29 cycles of 94°C for a minute, 37°C for 2 minutes, 72°C for 3 minutes, and the final incubation was at 72°C for 10 minutes.

The main product of the PCR reaction was a 413 bp fragment, which was gel purified. The purified fragment was ligated to the large fragment obtained from pBR322 digested with HindIII and SalI fragment, yielding the plasmid pBR322/S2d.

Ligation of the 413 bp HindIII-SalI S2 fragment with the 1.36 kb BamHI-HindIII fragment containing the ADH2/GAP promoter, and with the large BamHI-SalI fragment of the yeast vector pBS24.1 yielded recombinant vectors, which were cloned. Correct recombinant vectors were identified by the presence of a 1.77 kb fragment after digestion with BamHI and SalI. An expression vector constructed from the amplified sequence, and containing the sequence encoding S2 fused directly to the ADH2/GAP promoter is identified as pS2d#9.

### Example 4: Synthesis of HCV C Antigen

HCV antigen C22 is from the C domain. It comprises amino acids 1-122 of Figure 1.

The protocol for the construction of the expression vector encoding the C polypeptide and for its expression in yeast was analogous to that used for the expression of the C100 polypeptide, described supra, except for the following.

The template for the PCR reaction was pBR322/Ag30a which had been linearized with HindIII. Ag30 is a cDNA clone that encodes amino acids 1-122. The oligonucleotides used as primers for the amplification by PCR of the C encoding sequence were the following.
For the 5'-region of the C sequence: 
5′ GAG TGC AGC TTC AAA ACA AAA TGA GCA CGA
ATC CTA AAC CTC AAA AAA AAA AC 3′,
 and
for the 3'-region of the C sequence: 
5′ GAG TGC TCG TCG ACT CAT TAA CCC AAA TTG CGC
GAC CTA CGC CGG GGG TCT GT 3′.
 The primer for the 5'-region introduces a HindIII site into the amplified product, and the primer for the 3'-region introduces translation stop codons and a SalI site. The PCR was run for 29 cycles of 94°C for a minute, 37°C for 2 minutes, 72°C for 3 minutes, and the final incubation was at 72°C for 10 minutes.

The major product of PCR amplification is a 381 bp polynucleotide. Ligation of this fragment with the SalI-HindIII large SalI-HindIII fragment of pBR322 yielded the plasmid pBR322/C2.

Ligation of the 381 bp HindIII-SalI C coding fragment excised from pBR322/C2 with the 1.36 kb BamHI-HindIII fragment containing the ADH2/GAP promoter, and with the large BamHI-SalI fragment of the yeast vector pBS24.1 yielded recombinant vectors, which were cloned. Correct recombinant vectors were identified by the presence of a 1.74 kb fragment after digestion with BamHI and SalI. An expression vector constructed from the amplified sequence, and containing the sequence encoding C fused directly to the ADH2/GAP promoter is identified as pC22.

Analysis for expressed C polypeptide by the transformed cells was performed on total cell lysates and crude extracts prepared from single yeast colonies obtained from the Leu⁻ plates. The cell lysates and crude extracts were analyzed by electrophoresis on SDS polyacrylamide gels. The C polypeptide is expected to have 122 amino acids and by gel analysis the expressed polypeptide has a MWᵣ of approximately 13.6 Kd.

### Example 5: Synthesis of NS5 Polypeptide

This polypeptide contains sequence from the N-terminus of the NS5 domain. Specifically it includes amino acids 2054 to 2464 of Figure 1. The protocol for the construction of the expression vector encoding the NS5 polypeptide and for its expression were analogous to that used for the expression of C33c (see Example 1).

### Example 6: Radioimmunoassay (RIA) for Antibodies to HCV

The HCV antigens of Examples 1-5 were tested in an RIA format for their ability to detect antibodies to HCV in the serum of individuals clinically diagnosed as having HCV (Non-A, Non-B) and in serum from blood given by paid blood donors.

The RIA was based upon the procedure of Tsu and Herzenberg (1980) in SELECTED METHODS IN CELLULAR IMMUNOLOGY (W.H. Freeman & Co.), pp. 373-391. Generally, microtiter plates (Immulon 2, Removawell strips) are coated with purified HCV antigen. The coated plates are incubated with the serum samples or appropriate controls. During incubation, antibody, if present, is immunologically bound to the solid phase antigen. After removal of the unbound material and washing of the microtiter plates, complexes of human antibody-NANBV antigen are detected by incubation with ¹²⁵I-labeled sheep anti-human immunoglobulin. Unbound labeled antibody is removed by aspiration, and the plates are washed. The radioactivity in individual wells is determined; the amount of bound human anti-HCV antibody is proportional to the radioactivity in the well.

Specifically, one hundred microliter aliquots containing 0.1 to 0.5 micrograms of the HCV antigen in 0.125 M Na borate buffer, pH 8.3, 0.075 M NaCl (BBS) was added to each well of a microtiter plate (Dynatech Immulon 2 Removawell Strips). The plate was incubated at 4°C overnight in a humid chamber, after which, the antigen solution was removed and the wells washed 3 times with BBS containing 0.02% Triton X-100^{(TM)} (BBST). To prevent nonspecific binding, the wells were coated with bovine serum albumin (BSA) by addition of 100 microliters of a 5 mg/ml solution of BSA in BBS followed by incubation at room temperature for 1 hour; after this incubation the BSA solution was removed. The antigens in the coated wells were reacted with serum by adding 100 microliters of serum samples diluted 1:100 in 0.01M Na phosphate buffer, pH 7.2, 0.15 M NaCl (PBS) containing 10 mg/ml BSA, and incubating the serum containing wells for 1 hr at 37°C. After incubation, the serum samples were removed by aspiration, and the wells were washed 5 times with BBST. Antibody bound to the antigen was determined by the binding of ¹²⁵I-labeled F'(ab)₂ sheep anti-human IgG to the coated wells. Aliquots of 100 microliters of the labeled probe (specific activity 5-20 microcuries/microgram) were added to each well, and the plates were incubated at 37°C for 1 hour, followed by removal of excess probe by aspiration, and 5 washes with BBST. The amount of radioactivity bound in each well was determined by counting in a counter which detects gamma radiation.

Table 1 below presents the results of the tests on the serum from individuals diagnosed as having HCV.

**Table 1**

| INDIVIDUAL | ANTIGEN | | | | |
|---|---|---|---|---|---|
| | S2 | C22 | C100 | C33c | NS5 |
| CVH IVDA | P | P | P(+++) | P | P |
| CVH IVDA | P | P | P(+) | P | P |
| CVH IVDA | P | P | P(+) | P | P |
| CVH NOS | P | P | P | P | P |
| AVH NOS HS | N | N | N | N | N |
| AVH NOS HS | P | N | N | N | N |
| AVH NOS HS | P | N | N | N | N |
| AVH NOS HS | P/N | N | N | N | N |
| AVH PTVH | N | N | N | P/N | N |
| AVH NOS HS | N | N | N | N | N |
| AVH NOS | N | N | N | N | P |
| AVH PTVH | N | N | N | N | N |
| AVH IVDA | N | P | N | P | P |
| AVH PTVH | P | P/N | N | N | P |
| AVH NOS | N | P | N | N | N |
| AVH IVDA | N | P | N | P | P |
| AVH NOS HS | P/N | N | N | N | N |
| AVH PTVH | N | N | N | N | N |
| CVH IVDA | P | P | P | P | P |
| CVH IVDA | P | P | P | P | P |
| AVH NOS HS | N | N | N | N | N |
| CVH PTVH | P | P | N | N | N |
| AVH PTVH | P | N | P(+) | P(+++) | N |
| CVH PTVH | N | P | P | P | P |
| CVH NOS HS | P | P | P | P | N |
| CVH NOS | N | P | P/N | P | P |
| CVH IVDA | N | N | N | P | N |
| AVH IVDA | P | P | P | P | P |
| AVH IVDA | P | P | P | P | P |
| CVH IVDA | P | P | P | P | P |
| AVH IVDA | P/N | P | N | P | P |
| AVH IVDA | N | P | P | P | N |
| CVH PTVH | P | P/N | N | N | N |
| CVH NOS | N | N | N | N | N |
| CVH NOS | N | N | N | N | N |
| CVH IVDA | P | P | P | P | P |
| AVH IVDA | P | P | P | P | P |
| CVH PTVH | P | P | P | P | P |
| AVH PTVH? | N | P | P | P | P |
| AVH IVDA | N | P | N | P | N |
| AVH NOS | N | N | N | N | N |
| AVH NOS | N | N | N | N | N |
| CVH NOS | N | P | N | N | P |
| CVH NOS | P | P | N | N | N |
| CVH NOS HS | P | P | P | P | P |
| CVH PTVH | P | P | N | P | P |
| AVH nurse | P | P | N | N | N |
| AVH IVDA | P | P | P | P | N |
| AVH IVDA | N | P | P(+) | P(+++) | N |
| AVH nurse | P/N | P | N | N | N |
| AVH PTVH | P/N | P | P | N | P |
| AVH NOS | N | P/N | N | N | P |
| AVH NOS | N | P | N | P | N |
| AVH PTVH | P | P/N | N | N | N |
| AVH PTVH | N | P | N | P | P |
| AVH PTVH | P | P | P | P | P |
| AVH PTVH | N | P | N | N | P |
| CVH PTVH | P/N | P | P(+) | P(+++) | N |
| AVH PTVH | N | P/N | P(+) | P(+++) | P |
| AVH PTVH | P | (?) | P | N | P |
| CVH PTVH | N | P | N | P | P |
| CVH PTVH | N | P | P | P | P |
| CVH PTVH | N | N | N | N | N |
| AVH NOS | N | N | N | N | N |
| AVH nurse | P | P | N | N | N |
| CVH PTVH | N | P | N | N | P |
| AVH IVDA | N | P | N | P/N | N |
| CVH PTVH | P | P | P(+) | P(+++) | P |
| AVH NOS | P | P | N | N | N |
| AVH NOS | P/N | P | N | N | P |
| AVH PTVH | P/N | P | P | P | P |
| AVH NOS | N | P | P | P | P/N |
| AVH IVDA | N | P | N | N | P |
| AVH NOS | N | P/N | N | N | N |
| AVH NOS | P | P | N | N | P |
| AVH PTVH | N | P | P | P | P |
| crypto | P | P | P | P | P |
| CVH NOS | N | P | P | P | P |
| CVH NOS | N | N | N | N | N |
| AVH PTVH | N | P | P(+) | P(++) | N |
| AVH PTVH | N | P/N | P(+) | P(++) | P |
| AVH PTVH | N | P/N | P(+) | P(++) | P |
| CVH IVDA | P | P | P | P | P |
| CVH IVDA | P | P | P | P | P |
| CVH IVDA | P | P | P | P | P |
| CVH IVDA | P | P | P | P | P |
| AVH NOS | N | P | N | N | N |
| CVH IVDA | P | P | P | P | P/N |
| AVH IVDA | P | P | P | P | N |
| AVH NOS | P | P | N | N | N |
| AVH NOS | P | P | N | N | N |
| CVH PTVH | P | P | N | N | P/N |
| AVH PTVH | N | P | N | P | P |
| AVH NOS | N | N | N | N | N |
| AVH NOS | N | P | N | N | N |
| AVH NOS | P | N | N | N | N |
| CVH NOS | N | N | N | N | N |
| AVH NOS | N | P/N | N | N | N |
| AVH IVDA | N | P | P | P | P |
| crypto | N | P | N | N | P/N |
| crypto | P | P | P/N | P | P |
| AVH IVDA | N | N | P | P | N |
| AVH IVDA | N | P | P | P | N |
| AVH NOS | N | N | N | N | N |
| AVH NOS | N | N | N | N | N |
| CVH IVDA | P | P | P | P | P |
| CVH PTVH | N | N | N | N | N |
| CVH PTVH | P | P | P(+) | P(+++) | P |
| CVH PTVH | P | P | P(+) | P(+++) | P |
| CVH NOS | P/N | N | N | N | N |
| CVH NOS | N | N | N | N | N |
| CVH PTVH | P | P | P | P | P |
| CVH PTVH | P | P | P | P | P |
| CVH PTVH | P | P | P | P | P |
| AVH IVDA | N | P | P | P | P |
| CVH NOS | N | N | N | N | N |
| CVH NOS | N | N | N | N | N |
| CVH PTVH | P | P | P | P | P |
| AVH NOS | P | P | N | N | P/N |
| AVH NOS | N | P/N | N | N | N |
| CVH PTVH | P | P | N | N | P |
| CVH NOS | N | P/N | N | N | N |
| AVH NOS | N | P | N | N | N |
| AVH NOS | N | P | N | N | N |
| CVH PTVH | N | P | N | N | N |
| AVH IVDA | N | P | N | P | P |
| AVH NOS | P | N | N | N | N |
| CVH NOS | N | N | N | N | N |
| CVH NOS | N | N | N | N | N |
| CVH IVDA | P | P | P | P | P |
| CVH IVDA | P/N | P | P | P | P |
| CVH IVDA | P | P | P | P | P |
| CVH IVDA | N | P | P | P | P |
| AVH NOS | N | P | N | N | N |
| CVH IVDA | N | P | N | N | P |
| CVH IVDA | N | P | N | N | P |
| AVH PTVH | P | P | N | P | P |
| AVH PTVH | P | P | N | P | P |
| CVH NOS | N | P/N | N | N | P/N |
| CVH NOS | N | P | N | N | N |
| CVH NOS | N | N | N | N | N |
| CVH PTVH | P | P | P | P | P |
| CVH PTVH | P | P | P | P | P |
| CVH PTVH | P | P | P | P | P |
| AVH IVDA | N | P | N | N | P |
| AVH IVDA | N | P | P(++) | P(+) | P |
| CVH PTVH | P | P | P | P | P |
| AVH PTVH | N | P | P | P | P |
| CVH PTVH? | N | P | P | P | P |
| CVH PTVH? | P/N | P | P | P | P |
| CVH NOS HS | P | P | N | N | N |
| CVH IVDA | P | P | P | P | N |
| CVH PTVH | N | P | P | P | P |
| CVH PTVH | P | P | P | P | P/N |
| CVH NOS | P | P | P | P | P |
| CVH IVDA | P | P | P | P | P |
| CVH PTVH | P | P | P | P | N |
| CVH PTVH | P | P | P | P | P |
| CVH NOS | N | N | N | N | P/N |
| CVH NOS | N | P/N | N | N | P/N |
| CVH PTVH | P | P | P | P | P |
| CVH NOS | N | P | N | N | N |
| CVH NOS | N | N | N | N | N |
| CVH NOS | P | P | N | N | P/N |
| CVH NOS | N | N | N | N | N |
| CVH NOS HS | P | P | P | P | P |
| CVH NOS HS | P | P | P | P | P |
| CVH PTVH | N | N | N | N | N |
| AVH PTVH | N | P | P | P | P |
| AVH NOS | | | - | - | |
| CVH PTVH | N | P | P/N | P(+++) | N |
| crypto | P | P | P | P | P |
| crypto | P | P | P | P | P |
| crypto | N | P | N | N | N |
| crypto | N | P | P | P | P |
| CVH PTVH | P | P | P | P | P |
| crypto | N | N | N | N | N |
| crypto | N | P | N | N | P/N |
| crypto | N | P | N | N | P |
| crypto | P | P | P | P | P |
| crypto | N | P | N | P | N |
| crypto | | | - | - | |
| crypto | | | - | - | |
| CVH NOS | | | - | - | |
| AVH-IVDA | N | P | N | P(+) | P |
| AVH-IVDA | N | P/N | N | P(++) | N |

| | | | | | |
|---|---|---|---|---|---|
| AVH = acute viral hepatitis CVH = chronic viral hepatitis PTVH = post-transfusion viral hepatitis IVDA = intravenous drug abuser crypto = cryptogenic hepatitis NOS = non-obvious source P = positive N = negative | | | | | |

Per these results, no single antigen reacted with all sera. C22 and C33c were the most reactive and S2 reacted with some sera from some putative acute HCV cases with which no other antigen reacted. Based on these results, the combination of two antigens that would provide the greatest range of detection is C22 and C33c. If one wished to detect a maximum of acute infections, S2 would be included in the combination.

Table 2 below presents the results of the testing on the paid blood donors.

**Table 2**

| | Antigens | | | | |
|---|---|---|---|---|---|
| Donor | C100 | C33c | C22 | S2 | NS5 |
| 1 | N | N | N | N | N |
| 2 | N | N | N | N | N |
| 3 | P | P | P | P | P |
| 4 | N | N | N | N | N |
| 5 | N | N | N | N | N |
| 6 | N | N | N | N | N |
| 7 | N | N | N | N | N |
| 8 | N | N | N | N | N |
| 9 | N | N | N | N | N |
| 10 | N | N | N | N | N |
| 11 | N | N | N | N | N |
| 12 | N | N | N | N | N |
| 13 | N | N | N | N | N |
| 14 | N | N | N | N | N |
| 15 | N | N | N | N | N |
| 16 | N | N | N | N | N |
| 17 | N | N | N | N | N |
| 18 | P | P | P | P | P |
| 19 | P | P | N | P | P |
| 20 | P | P | N | P | P |
| 21 | N | N | N | N | N |
| 22 | N | P | P | N | P |
| 23 | P | P | P | P | P |
| 24 | N | N | N | N | N |
| 25 | N | N | N | N | N |
| 26 | N | N | N | N | N |
| 27 | N | N | N | N | N |
| 28 | N | N | N | N | N |
| 29 | N | N | N | N | N |
| 30 | N | N | N | N | N |
| 31 | P | P | P | N | P |
| 32 | N | N | N | N | N |
| 33 | N | N | N | N | N |
| 34 | N | N | N | N | P |
| 35 | N | N | P | N | P |
| 36 | N | N | N | N | N |
| 37 | N | N | N | N | N |
| 38 | N | N | N | N | N |
| 39 | N | N | N | N | N |
| 40 | N | N | N | N | N |
| 41 | N | N | N | N | P |
| 42 | N | N | N | N | N |
| 43 | N | N | N | N | N |
| 44 | N | N | N | N | N |
| 45 | N | N | N | N | N |
| 46 | N | N | N | N | N |
| 47 | P | P | N | N | P |
| 48 | N | N | N | N | N |
| 49 | N | N | N | N | N |
| 50 | N | N | N | N | N |
| 51 | N | P | P | N | P |
| 52 | N | N | N | N | N |
| 53 | N | P | P | N | P |
| 54 | P | P | P | P | N |
| 55 | N | N | N | N | N |
| 56 | N | N | N | N | N |
| 57 | N | N | N | N | N |
| 58 | N | N | N | N | N |
| 59 | N | N | N | N | N |
| 60 | N | N | N | N | N |
| 61 | N | N | N | N | N |
| 62 | N | N | N | N | N |
| 63 | N | N | N | N | N |
| 64 | N | N | N | N | N |
| 65 | N | N | N | N | N |
| 66 | N | N | N | N | N |
| 67 | N | N | N | N | N |
| 68 | N | N | N | N | N |
| 69 | N | N | N | N | N |
| 70 | P | P | P | P | P |
| 71 | N | N | N | N | N |
| 72 | N | N | N | N | N |
| 73 | P | P | P | P | N |
| 74 | N | N | N | N | N |
| 75 | N | N | N | N | N |
| 76 | N | N | N | N | P |
| 77 | N | N | N | N | N |
| 78 | N | N | N | N | N |
| 79 | N | N | N | N | N |
| 80 | N | N | N | N | N |
| 81 | N | N | N | N | N |
| 82 | N | N | N | N | N |
| 83 | P | P | N | N | N |
| 84 | N | N | P | N | N |
| 85 | N | N | N | N | N |
| 86 | P | P | P | P | N |
| 87 | N | N | N | N | N |
| 88 | N | N | N | N | N |
| 89 | P | P | P | P | P |
| 90 | P | P | P | P | N |
| 91 | N | N | N | N | P |
| 92 | P | P | P | N | N |
| 93 | N | N | N | N | N |
| 94 | N | N | N | N | N |
| 95 | N | N | N | N | N |
| 96 | N | N | N | N | N |
| 97 | N | N | N | N | N |
| 98 | N | P | P | P | P |
| 99 | P | P | P | P | P |
| 100 | N | N | N | N | N |
| 101 | P | P | P | P | P |
| 102 | N | N | N | N | N |
| 103 | N | N | N | N | N |
| 104 | | N | N | N | N |
| 105 | P | P | P | P | N |
| 106 | N | N | N | N | N |
| 107 | N | N | N | N | N |
| 108 | N | N | N | N | N |
| 109 | P | P | P | P | P |
| 110 | P | P | P | N | P |
| 111 | P | P | P | N | P |
| 112 | N | N | N | N | N |
| 113 | P | P | P | P | P |
| 114 | N | N | N | N | N |
| 115 | N | N | N | N | N |
| 116 | P | P | P | P | P |
| 117 | N | N | N | N | N |
| 118 | N | N | N | N | N |
| 119 | N | N | N | N | N |
| 120 | P | P | P | P | P |
| 121 | N | N | N | N | N |
| 122 | N | P | P | N | P |
| 123 | N | N | N | N | N |
| 124 | N | N | N | N | N |
| 125 | N | N | N | N | N |
| 126 | P | N | N | N | N |
| 127 | N | N | N | N | N |
| 128 | N | N | N | N | N |
| 129 | N | N | N | N | N |
| 130 | P | P | P | P | N |
| 131 | N | N | N | N | P |
| 132 | N | N | N | N | N |
| 133 | N | N | N | N | N |
| 134 | N | N | N | N | N |
| 135 | N | N | N | N | N |
| 136 | N | N | N | N | N |
| 137 | N | N | N | N | N |
| 138 | N | N | N | N | N |
| 139 | N | N | N | N | N |
| 140 | P | N | N | N | N |
| 141 | P | N | P | P | P |
| 142 | N | N | N | N | N |
| 143 | N | N | N | N | N |
| 144 | N | N | N | N | N |
| 145 | N | N | N | N | N |
| 146 | N | N | N | N | N |
| 147 | N | N | N | N | N |
| 148 | N | N | N | N | N |
| 149 | N | N | N | N | N |
| 150 | N | N | N | N | N |
| 151 | N | N | N | N | N |
| 152 | N | N | N | N | N |
| 153 | N | N | N | N | N |
| 154 | P | P | P | P | P |
| 155 | N | N | N | N | N |
| 156 | N | N | N | N | N |
| 157 | N | N | N | N | N |
| 158 | N | N | N | N | N |
| 159 | N | N | N | N | N |
| 160 | N | N | N | N | N |
| 161 | P | P | P | P | P |
| 162 | N | N | N | N | N |
| 163 | N | N | N | N | N |
| 164 | P | P | P | N | P |
| 165 | N | N | N | N | N |
| 166 | P | P | P | N | P |
| 167 | N | N | N | N | N |
| 168 | N | N | N | N | N |
| 169 | N | N | N | N | N |
| 170 | N | N | N | N | N |
| 171 | N | N | N | N | N |
| 172 | N | N | N | N | N |
| 173 | N | N | N | N | N |
| 174 | N | N | N | N | N |
| 175 | N | N | N | N | N |
| 176 | N | N | N | N | N |
| 177 | N | N | N | N | P |
| 178 | N | N | N | N | N |
| 179 | N | N | N | N | N |
| 180 | N | N | N | N | N |
| 181 | N | N | N | N | N |
| 182 | N | N | N | N | N |
| 183 | P | P | P | P | P |
| 184 | N | N | N | N | N |
| 185 | N | N | N | N | N |
| 186 | N | N | N | N | N |
| 187 | N | N | N | N | N |
| 188 | N | P | P | N | N |
| 189 | N | N | N | N | N |
| 190 | N | N | N | N | N |
| 191 | N | N | N | N | N |
| 192 | N | N | N | N | N |
| 193 | N | N | N | N | N |
| 194 | N | N | N | N | N |
| 195 | N | N | N | N | N |
| 196 | N | N | N | N | N |
| 197 | N | N | N | N | P |
| 198 | P | P | P | N | N |
| 199 | N | N | N | N | P |
| 200 | P | P | P | P | N |

The results on the paid donors generally confirms the results from the sera of infected individuals.

### Example 7: ELISA Determinations of HCV Antibodies Using Combination of HCV Antigens

Plates coated with a combination of C22 and C33c antigens are prepared as follows. A solution containing coating buffer (50mM Na Borate, pH 9.0), 21 ml/plate, BSA (25 micrograms/ml), C22 and C33c (2.50 micrograms/ml each) is prepared just prior to addition to the Removeawell Immulon I plates (Dynatech Corp.). After mixing for 5 minutes, 0.2ml/well of the solution is added to the plates, they are covered and incubated for 2 hours at 37°C, after which the solution is removed by aspiration. The wells are washed once with 400 microliters wash buffer (100 mM sodium phosphate, pH 7.4, 140 mM sodium chloride, 0.1% (W/V) casein, 1% (W/V) Triton x-100^{(TM)}, 0.01% (W/V) Thimerosal). After removal of the wash solution, 200 microliters/well of Postcoat solution (10 mM sodium phosphate, pH 7.2, 150 mM sodium chloride, 0.1% (w/v) casein, 3% sucrose and 2 mM phenylmethylsulfonylfluoride (PMSF)) is added, the plates are loosely covered to prevent evaporation, and are allowed to stand at room temperature for 30 minutes. The wells are then aspirated to remove the solution, and lyophilized dry overnight, without shelf heating. The prepared plates may be stored at 2-8°C in sealed aluminum pouches with dessicant (3 g Sorb-it™ packs).

In order to perform the ELISA determination, 20 microliters of serum sample or control sample is added to a well containing 200 microliters of sample diluent (100 mM sodium phosphate, pH 7.4, 500 mM sodium chloride, 1 mM EDTA, 0.1% (W/V) Casein, 0.01% (W/V) Thimerosal, 1% (W/V) Triton X-100^{(TM)}, 100 micrograms/ml yeast extract). The plates are sealed, and are incubated at 37°C for two hours, after which the solution is removed by aspiration, and the wells are washed three times with 400 microliters of wash buffer (phosphate buffered saline (PBS) containing 0.05% Tween 20^{(TM)}). The washed wells are treated with 200 microliters of mouse anti-human IgG-horse radish peroxidase (HRP) conjugate contained in a solution of Ortho conjugate diluent (10 mM sodium phosphate, pH 7.2, 150 mM sodium chloride, 50% (v/v) fetal bovine serum, 1% (V/V) heat treated horse serum, 1 mM K₃Fe(CN)₆, 0.05% (W/V) Tween 20, 0.02% (W/V) Thimerosal). Treatment is for 1 hour at 37°C, the solution is removed by aspiration, and the wells are washed three times with 400 ml wash buffer, which is also removed by aspiration. To determine the amount of bound enzyme conjugate, 200 microliters of substrate solution (10 mg O-phenylenediamine dihydrochloride per 5 ml of Developer solution) is added. Developer solution contains 50 mM sodium citrate adjusted to pH 5.1 with phosphoric acid, and 0.6 microliters/ml of 30% H₂O₂. The plates containing the substrate solution are incubated in the dark for 30 minutes at room temperature, the reactions are stopped by the addition of 50 microliters/ml 4N sulfuric acid, and the ODs determined.

In a similar manner, ELISAs using fusion proteins of C22 and C33c, and C22, C33c, and S2 and combinations of C22 and C100, C22 and S2, C22 and an NS5 antigen, C22, C33c, and S2, and C22, C100, and S2 may be carried out.

Modifications of the above-described modes for carrying out the invention that are obvious to those of skill in the fields of molecular biology, immunology, and related fields are intended to be within the scope of the following claims.

## Claims (Claims for the following Contracting State(s): AT, BE, CH, LI, DE, DK, FR, GB, GR, IT, LU, NL, SE)

1. A combination of hepatitis.C virus (HCV) antigens in one or more polypeptides made by chemical synthesis or recombinant expression, comprising:
(a) a first HCV antigen comprising an epitope from the C domain of the HCV 1 polyprotein shown in Figure 1; and
(b) a second HCV antigen selected from
(i) an HCV antigen comprising an epitope from the S domain of the HCV 1 polyprotein shown in Figure 1;
(ii) an HCV antigen comprising an epitope from the NS3 domain of the HCV 1 polyprotein shown in Figure 1;
(iii)an HCV antigen comprising an epitope from the NS4 domain of the HCV 1 polyprotein shown in Figure 1; and
(iv) an HCV antigen comprising an epitope from the NS5 domain of the HCV 1 polyprotein shown in Figure 1;
with the proviso that the combination is not the peptide p1 with C100-3, the peptide p35 with C100-3, the peptide p99 with C100-3, or a peptide having amino acids 9 to 177 of the HCV 1 polyprotein.

2. A combination according to claim 1 wherein said second antigen comprises an epitope from the S domain of the HCV 1 polyprotein shown in Figure 1 and which combination further comprises at least one additional HCV antigen selected from
(i) an HCV antigen comprising an epitope from the NS3 domain of the HCV 1 polyprotein shown in Figure 1;
(ii) an HCV antigen comprising an epitope from the NS4 domain of the HCV 1 polyprotein shown in Figure 1; and
(iii) an HCV antigen comprising an epitope from the NS5 domain of the HCV 1 polyprotein shown in Figure 1.

3. A combination according to claim 2 wherein the additional antigen is from the NS3 domain of the HCV 1 polyprotein shown in Figure 1.

4. A combination according to claim 3 wherein the first antigen is C22 and the second HCV antigen is C33c.

5. A combination according to claim 2, wherein the additional antigen is from the NS4 domain of the HCV 1 polyprotein shown in Figure 1.

6. A combination according to claim 5 wherein the first antigen is C22 and the second HCV antigen is C100.

7. A combination according to any one of claims 1 to 6 wherein the antigen from the S domain of the HCV 1 polyprotein shown in Figure 1 is antigen S2.

8. A combination according to any one of claims 1 to 7 wherein the combination is in the form of a fusion polypeptide.

9. A combination according to any one of claims 1 to 8 wherein the combination is in the form of said first HCV antigen and said additional antigens bound to a common solid matrix.

10. A combination according to claim 9 wherein the solid matrix is the surface of a microtiter plate well, a bead or a dipstick.

11. A combination according to any one of claims 1 to 7 wherein the combination is immobilized on the surface of a solid matrix suitable for detection of HCV by immunoassay.

12. A method for detecting antibodies to HCV in a mammalian body component suspected of containing said antibodies comprising contacting said body component with a combination of antigens according to any one of claims 1 to 11 under conditions that permit antibody antigen reaction and detecting the presence of immune complexes of said antibodies and said antigens.

13. A kit for carrying out an assay for detecting antibodies to hepatitis C antigen (HCV) in a mammalian body component suspected of containing said antibodies comprising in packaged combination:
(a) the combination according to any one of claims 1 to 11;
(b) standard control reagents; and
(c) instructions for carrying out the assay.

## Claims (Claims for the following Contracting State(s): ES)

1. A method for detecting antibodies to hepatitis C virus (HCV) in a mammalian body component suspected of containing said antibodies comprising contacting said body component with a combination of HCV antigens under conditions that permit antibody-antigen reaction and detecting the presence of immune complexes of said antibodies and said antigens, wherein the antigens are in one or more polypeptides made by chemical synthesis or recombinant expression, immobilized on the surface of a solid matrix suitable for detection of HCV by immunoassay, comprising:
(a) a first HCV antigen comprising an epitope from the C domain of the HCV 1 polyprotein shown in Figure 1; and
(b) a second HCV antigen selected from
(i) an HCV antigen comprising an epitope from the S domain of the HCV 1 polyprotein shown in Figure 1;
(ii) an HCV antigen comprising an epitope from the NS3 domain of the HCV 1 polyprotein shown in Figure 1;
(iii) an HCV antigen comprising an epitope from the NS4 domain of the HCV 1 polyprotein shown in Figure 1; and
(iv) an HCV antigen comprising an epitope from the NS5 domain of the HCV 1 polyprotein shown in Figure 1;
with the proviso that the combination is not the peptide p1 with C100-3, the peptide p35 with C100-3, the peptide p99 with C100-3, or a peptide having amino acids 9 to 177 of the HCV 1 polyprotein.

2. A method according to claim 1 wherein said second antigen comprises an epitope from the S domain of the HCV 1 polyprotein shown in Figure 1 and which combination further comprises at least one additional HCV antigen selected from
(i) an HCV antigen comprising an epitope from the NS3 domain of the HCV 1 polyprotein shown in Figure 1;
(ii) an HCV antigen comprising an epitope from the NS4 domain of the HCV 1 polyprotein shown in Figure 1; and
(iii) an HCV antigen comprising an epitope from the NS5 domain of the HCV 1 polyprotein shown in Figure 1.

3. A method according to claim 2 wherein the additional antigen is from the NS3 domain of the HCV 1 polyprotein shown in Figure 1.

4. A method according to claim 2 wherein the first HCV antigen is C22 and the additional HCV antigen is C33c.

5. A method according to claim 2 wherein the additional antigen is from the NS4 domain of the HCV 1 polyprotein shown in Figure 1.

6. A method according to claim 5 wherein the first antigen is C22 and the second HCV antigen is C100.

7. A method according to any one of claims 1 or 6 wherein the antigen from the S domain of the HCV 1 polyprotein shown in Figure 1 is antigen S2.

8. A method according to any one of claims 1 to 7 wherein the combination is in the form of a fusion polypeptide.

9. A method according to any one of claims 1 to 8 wherein the combination is in the form of said first HCV antigen and said additional antigens bound to a common solid matrix.

10. A method according to claim 8 wherein the solid matrix is the surface of a microtiter plate well, a bead or a dipstick.

11. A combination of hepatitis C virus (HCV) antigens in one or more polypeptides made by chemical synthesis or recombinant expression, comprising:
(a) a first HCV antigen comprising an epitope from the C domain of the HCV 1 polyprotein shown in Figure 1; and
(b) a second HCV antigen selected from
(i) an HCV antigen comprising an epitope from the S domain of the HCV 1 polyprotein shown in Figure 1;
(ii) an HCV antigen comprising an epitope from the NS3 domain of the HCV 1 polyprotein shown in Figure 1;
(iii)an HCV antigen comprising an epitope from the NS4 domain of the HCV 1 polyprotein shown in Figure 1; and
(iv) an HCV antigen comprising an epitope from the NS5 domain of the HCV 1 polyprotein shown in Figure 1;
with the proviso that the combination is not the peptide p1 with C100-3, the peptide p35 with C100-3, the peptide p99 with C100-3, or a peptide having amino acids 9 to 177 of the HCV 1 polyprotein.

12. A combination according to claim 11 wherein said second antigen comprises an epitope from the S domain of the HCV 1 polyprotein shown in Figure 1 and which combination further comprises at least one additional HCV antigen selected from
(i) an HCV antigen comprising an epitope from the NS3 domain of the HCV 1 polyprotein shown in Figure 1;
(ii) an HCV antigen comprising an epitope from the NS4 domain of the HCV 1 polyprotein shown in Figure 1; and
(iii) an HCV antigen comprising an epitope from the NS5 domain of the HCV 1 polyprotein shown in Figure 1.

13. A combination according to claim 12 wherein the additional antigen is from the NS3 domain of the HCV 1 polyprotein shown in Figure 1.

14. A combination according to claim 13 wherein the first HCV antigen is C22 and the additional HCV antigen is C33c.

15. A combination according to claim 14, wherein the additional antigen is from the NS4 domain of the HCV 1 polyprotein shown in Figure 1.

16. A combination according to claim 15 wherein the first antigen is C22 and the second HCV antigen is C100.

17. A combination according to any one of claims 11 to 16 wherein the antigen from the S domain of the HCV 1 polyprotein shown in Figure 1 is antigen S2.

18. A combination according to any one of claims 11 to 17 wherein the combination is in the form of a fusion polypeptide.

19. A combination according to any one of claims 11 to 18 wherein the combination is in the form of said first HCV antigen and said additional antigens bound to a common solid matrix.

20. A combination according to claim 19 wherein the solid matrix is the surface of a microtiter plate well, a bead or a dipstick.

21. A combination according to any one of claims 11 to 20 wherein the combination is immobilized on the surface of a solid matrix suitable for detection of HCV by immunoassay.

22. A kit for carrying out an assay for detecting antibodies to hepatitis C antigen (HCV) in a mammalian body component suspected of containing said antibodies comprising in packaged combination:
(a) the combination according to any one of claims 11 to 21;
(b) standard control reagents; and
(c) instructions for carrying out the assay.

## Patentansprüche (Patentansprüche für folgende(n) Vertragsstaat(en): AT, BE, CH, LI, DE, DK, FR, GB, GR, IT, LU, NL, SE)

1. Kombination von Hepatitis-C-Virus (HCV)-Antigenen in einem oder in mehreren Polypeptiden, das/die durch chemische Synthese oder durch rekombinante Expression hergestellt wurde/wurden, umfassend:
(a) ein erstes HCV-Antigen, das ein Epitop aus der C-Domäne des in Figur 1 gezeigten HCV-1-Polyproteins umfasst; und
(b) ein zweites HCV-Antigen, ausgewählt aus
(i) einem HCV-Antigen, das ein Epitop aus der S-Domäne des in Figur 1 gezeigten HCV-1-Polyproteins umfasst;
(ii) einem HCV-Antigen, das ein Epitop aus der NS3-Domäne des in Figur 1 gezeigten HCV-1-Polyproteins umfasst;
(iii) einem HCV-Antigen, das ein Epitop aus der NS4-Domäne des in Figur 1 gezeigten HCV-1-Polyproteins umfasst; und
(iv) einem HCV-Antigen, das ein Epitop aus der NS5-Domäne des in Figur 1 gezeigten HCV-1-Polyproteins umfasst;
mit der Maßgabe, dass die Kombination nicht das Peptid p1 mit C100-3, das Peptid p35 mit C100-3, das Peptid p99 mit C100-3 oder ein Peptid mit den Aminosäuren 9 bis 177 des HCV-1-Polyproteins ist.

2. Kombination nach Anspruch 1, wobei das zweite Antigen ein Epitop aus der S-Domäne des in Figur 1 gezeigten HCV-1-Polyproteins umfasst, und wobei die Kombination ferner mindestens ein zusätzliches HCV-Antigen umfasst, ausgewählt aus:
(i) einem HCV-Antigen, das ein Epitop aus der NS3-Domäne des in Figur 1 gezeigten HCV-1-Polyproteins umfasst;
(ii) einem HCV-Antigen, das ein Epitop aus der NS4-Domäne des in Figur 1 gezeigten HCV-1-Polyproteins umfasst; und
(iii) einem HCV-Antigen, das ein Epitop aus der NS5-Domäne des in Figur 1 gezeigten HCV-1-Polyproteins umfasst.

3. Kombination nach Anspruch 2, wobei das zusätzliche Antigen aus der NS3-Domäne des in Figur 1 gezeigten HCV-1-Polyproteins ist.

4. Kombination nach Anspruch 3, wobei das erste Antigen C22 ist und das zweite HCV-Antigen C33c ist.

5. Kombination nach Anspruch 2, wobei das zusätzliche Antigen aus der NS4-Domäne des in Figur 1 gezeigten HCV-1-Polyproteins ist.

6. Kombination nach Anspruch 5, wobei das erste Antigen C22 ist und das zweite HCV-Antigen C100 ist.

7. Kombination nach einem der Ansprüche 1 bis 6, wobei das Antigen aus der S-Domäne des in Figur 1 gezeigten HCV-1-Polyproteins das Antigen S2 ist.

8. Kombination nach einem der Ansprüche 1 bis 7, wobei die Kombination in Form eines Fusionspolypeptids vorliegt.

9. Kombination nach einem der Ansprüche 1 bis 8, wobei die Kombination in einer Form vorliegt, bei der das erste HCV-Antigen und die zusätzlichen Antigene an eine gemeinsame feste Matrix gebunden sind.

10. Kombination nach Anspruch 9, wobei die feste Matrix die Oberfläche einer Mikrotiterplattenvertiefung, eines Beads oder eines Eintauchstäbchens (dipstick) ist.

11. Kombination nach einem der Ansprüche 1 bis 7, wobei die Kombination auf der Oberfläche einer festen Matrix, die zum Nachweis von HCV durch Immunassay geeignet ist, immobilisiert ist.

12. Verfahren zum Nachweis von Antikörpern gegen HCV in einem Bestandteil des Säugetierkörpers, von dem angenommen wird, dass er diese Antikörper enthält, bei dem man den Bestandteil des Körpers mit einer Kombination von Antigenen nach einem der Ansprüche 1 bis 11 unter Bedingungen in Kontakt bringt, die eine Antikörper-Antigen-Reaktion erlauben, und das Vorhandensein von Immunkomplexen aus den Antikörpern und den Antigenen nachweist.

13. Kit zur Durchführung eines Assays zum Nachweis von Antikörpern gegen Hepatitis-C-Antigen in einem Bestandteil des Säugetierkörpers, von dem angenommen wird, dass er die Antikörper enthält, umfassend:
(a) die Kombination nach einem der Ansprüche 1 bis 11;
(b) Standardkontrollreagenzien; und
(c) Anweisungen zur Durchführung des Assays
in verpackter Kombination.

## Patentansprüche (Patentansprüche für folgende(n) Vertragsstaat(en): ES)

1. Verfahren zum Nachweis von Antikörpern gegen Hepatitis-C-Virus (HCV) in einem Bestandteil eines Säugetierkörpers, von dem angenommen wird, dass er diese Antikörper enthält, bei dem man den Bestandteil des Körpers mit einer Kombination von HCV-Antigenen unter Bedingungen in Kontakt bringt, die eine Antikörper-Antigen-Reaktion erlauben, und das Vorhandensein von Immunkomplexen aus den Antikörpern und den Antigenen nachweist, wobei die Antigene in einem oder in mehreren Polypeptiden vorliegen, das/die durch chemische Synthese oder durch rekombinante Expression hergestellt wurde/wurden und auf der Oberfläche einer festen Matrix, die zum Nachweis von HCV durch Immunassay geeignet ist, immobilisiert wurde/wurden, umfassend:
(a) ein erstes HCV-Antigen das ein Epitop aus der C-Domäne des in Figur 1 gezeigten HCV-1-Polyproteins umfasst; und
(b) ein zweites HCV-Antigen, ausgewählt aus
(i) einem HCV-Antigen, das ein Epitop aus der S-Domäne des in Figur 1 gezeigten HCV-1-Polyproteins umfasst;
(ii) einem HCV-Antigen, das ein Epitop aus der NS3-Domäne des in Figur 1 gezeigten HCV-1-Polyproteins umfasst;
(iii) einem HCV-Antigen, das ein Epitop aus der NS4-Domäne des in Figur 1 gezeigten HCV-1-Polyproteins umfasst; und
(iv) einem HCV-Antigen, das ein Epitop aus der NS5-Domäne des in Figur 1 gezeigten HCV-1-Polyproteins umfasst;
mit der Maßgabe, dass die Kombination nicht das Peptid p1 mit C100-3, das Peptid p35 mit C100-3, das Peptid p99 mit C100-3 oder ein Peptid mit den Aminosäuren 9 bis 177 des HCV-1-Polyproteins ist.

2. Verfahren nach Anspruch 1, wobei das zweite Antigen ein Epitop aus der S-Domäne des in Figur 1 gezeigten HCV-1-Polyproteins umfasst, und wobei die Kombination ferner mindestens ein zusätzliches HCV-Antigen umfasst, ausgewählt aus:
(i) einem HCV-Antigen, das ein Epitop aus der NS3-Domäne des in Figur 1 gezeigten HCV-1-Polyproteins umfasst;
(ii) einem HCV-Antigen, das ein Epitop aus der NS4-Domäne des in Figur 1 gezeigten HCV-1-Polyproteins umfasst; und
(iii) einem HCV-Antigen, das ein Epitop aus der NS5-Domäne des in Figur 1 gezeigten HCV-1-Polyproteins umfasst.

3. Verfahren nach Anspruch 2, wobei das zusätzliche Antigen aus der NS3-Domäne des in Figur 1 gezeigten HCV-1-Polyproteins ist.

4. Verfahren nach Anspruch 2, wobei das erste HCV-Antigen C22 ist und das zusätzliche HCV-Antigen C33c ist.

5. Verfahren nach Anspruch 2, wobei das zusätzliche Antigen aus der NS4-Domäne des in Figur 1 gezeigten HCV-1-Polyproteins ist.

6. Verfahren nach Anspruch 5, wobei das erste HCV-Antigen C22 ist und das zweite HCV-Antigen C100 ist.

7. Verfahren nach einem der Ansprüche 1 bis 6, wobei das Antigen aus der S-Domäne des in Figur 1 gezeigten HCV-1-Polyproteins das Antigen S2 ist.

8. Verfahren nach einem der Ansprüche 1 bis 7, wobei die Kombination in Form eines Fusionspolypeptids vorliegt.

9. Verfahren nach einem der Ansprüche 1 bis 8, wobei die Kombination in einer Form vorliegt, bei der das erste HCV-Antigen und die zusätzlichen Antigene an eine gemeinsame feste Matrix gebunden sind.

10. Verfahren nach Anspruch 8, wobei die feste Matrix die Oberfläche einer Mikrotiterplattenvertiefung, eines Beads oder eines Eintauchstäbchens (dipstick) ist.

11. Kombination von Hepatitis-C-Virus (HCV)-Antigenen in einem oder in mehreren Polypeptiden, das/die durch chemische Synthese oder durch rekombinante Expression hergestellt wurde/wurden, umfassend:
(a) ein erstes HCV-Antigen, das ein Epitop aus der C-Domäne des in Figur 1 gezeigten HCV-1-Polyproteins umfasst; und
(b) ein zweites HCV-Antigen, ausgewählt aus
(i) einem HCV-Antigen, das ein Epitop aus der S-Domäne des in Figur 1 gezeigten HCV-1-Polyproteins umfasst;
(ii) einem HCV-Antigen, das ein Epitop aus der NS3-Domäne des in Figur 1 gezeigten HCV-1-Polyproteins umfasst;
(iii) einem HCV-Antigen, das ein Epitop aus der NS4-Domäne des in Figur 1 gezeigten HCV-1-Polyproteins umfasst; und
(iv) einem HCV-Antigen, das ein Epitop aus der NS5-Domäne des in Figur 1 gezeigten HCV-1-Polyproteins umfasst;
mit der Maßgabe, dass die Kombination nicht das Peptid p1 mit C100-3, das Peptid p35 mit C100-3, das Peptid p99 mit C100-3 oder ein Peptid mit den Aminosäuren 9 bis 177 des HCV-1-Polyproteins ist.

12. Kombination nach Anspruch 11, wobei das zweite Antigen ein Epitop aus der S-Domäne des in Figur 1 gezeigten HCV-1-Polyproteins umfasst, und wobei die Kombination ferner mindestens ein zusätzliches HCV-Antigen umfasst, ausgewählt aus:
(i) einem HCV-Antigen, das ein Epitop aus der NS3-Domäne des in Figur 1 gezeigten HCV-1-Polyproteins umfasst;
(ii) einem HCV-Antigen, das ein Epitop aus der NS4-Domäne des in Figur 1 gezeigten HCV-1-Polyproteins umfasst; und
(iii) einem HCV-Antigen, das ein Epitop aus der NS5-Domäne des in Figur 1 gezeigten HCV-1-Polyproteins umfasst.

13. Kombination nach Anspruch 12, wobei das zusätzliche Antigen aus der NS3-Domäne des in Figur 1 gezeigten HCV-1-Polyproteins ist.

14. Kombination nach Anspruch 13, wobei das erste HCV-Antigen C22 ist und das zusätzliche HCV-Antigen C33c ist.

15. Kombination nach Anspruch 14, wobei das zusätzliche Antigen aus der NS4-Domäne des in Figur 1 gezeigten HCV-1-Polyproteins ist.

16. Kombination nach Anspruch 15, wobei das erste Antigen C22 ist und das zweite HCV-Antigen C100 ist.

17. Kombination nach einem der Ansprüche 11 bis 16, bei der das Antigen aus der S-Domäne des in Figur 1 gezeigten HCV-1-Polyproteins das Antigen S2 ist.

18. Kombination nach einem der Ansprüche 11 bis 17, wobei die Kombination in Form eines Fusionspolypeptids vorliegt.

19. Kombination nach einem der Ansprüche 11 bis 18, wobei die Kombination in einer Form vorliegt, bei der das erste HCV-Antigen und die zusätzlichen Antigene an eine gemeinsame feste Matrix gebunden sind.

20. Kombination nach Anspruch 19, wobei die feste Matrix die Oberfläche einer Mikrotiterplattenvertiefung, eines Beads oder eines Eintauchstäbchens (dipstick) ist.

21. Kombination nach einem der Ansprüche 11 bis 20, wobei die Kombination auf der Oberfläche einer festen Matrix, zum Nachweis von HCV durch einen Immunassay geeignet ist, immobilisiert ist.

22. Kit zur Durchführung eines Assays zum Nachweis von Antikörpern gegen Hepatitis-C-Antigen in einem Bestandteil des Säugetierkörpers, von dem angenommen wird, dass er die Antikörper enthält, umfassend:
(a) die Kombination nach einem der Ansprüche 11 bis 21;
(b) Standardkontrollreagenzien; und
(c) Anweisungen zur Durchführung des Assays
in verpackter Kombination.

## Revendications (Revendications pour l'(les) Etat(s) contractant(s) suivant(s): AT, BE, CH, LI, DE, DK, FR, GB, GR, IT, LU, NL, SE)

1. Combinaison d'antigènes du virus de l'hépatite C (HCV) en un ou plusieurs polypeptides obtenus par synthèse chimique ou expression recombinante, comprenant :
(a) un premier antigène de HCV comprenant un épitope du domaine C de la polyprotéine de HCV 1 représentée dans la figure 1 ; et
(b) un deuxième antigène de HCV choisi parmi
(i) un antigène de HCV comprenant un épitope du domaine S de la polyprotéine de HCV 1 représentée dans la figure 1 ;
(ii) un antigène de HCV comprenant un épitope du domaine NS3 de la polyprotéine de HCV 1 représentée dans la figure 1 ;
(iii) un antigène de HCV comprenant un épitope du domaine NS4 de la polyprotéine de HCV 1 représentée dans la figure 1 ; et
(iv) un antigène de HCV comprenant un épitope du domaine NS5 de la polyprotéine de HCV 1 représentée dans la figure 1;
sous réserve que la combinaison ne soit pas le peptide p1 avec C100-3, le peptide p35 avec C100-3, le peptide p99 avec C100-3 ou un peptide ayant les acides aminés 9 à 177 de la polyprotéine de HCV 1.

2. Combinaison selon la revendication 1, dans laquelle ledit deuxième antigène comprend un épitope du domaine S de la polyprotéine de HCV 1 représentée dans la figure 1, et laquelle combinaison comprend en outre au moins un antigène de HCV supplémentaire choisi parmi
(i) un antigène de HCV comprenant un épitope du domaine NS3 de la polyprotéine de HCV 1 représentée dans la figure 1 ;
(ii) un antigène de HCV comprenant un épitope du domaine NS4 de la polyprotéine de HCV 1 représentée dans la figure 1 ; et
(iii) un antigène de HCV comprenant un épitope du domaine NS5 de la polyprotéine de HCV 1 représentée dans la figure 1.

3. Combinaison selon la revendication 2, dans laquelle l'antigène supplémentaire provient du domaine NS3 de la polyprotéine de HCV 1 représentée dans la figure 1.

4. Combinaison selon une quelconque revendication 3, dans laquelle le premier antigène est C22 et le deuxième antigène de HCV est C33c.

5. Combinaison selon la revendication 2, dans laquelle l'antigène supplémentaire provient du domaine NS4 de la polyprotéine de HCV 1 représentée dans la figure 1.

6. Combinaison selon une quelconque revendication 5, dans laquelle le premier antigène est C22 et le deuxième antigène de HCV est C100.

7. Combinaison selon l'une quelconque des revendications 1 à 6, dans laquelle l'antigène du domaine S de la polyprotéine de HCV 1 représentée dans la figure 1 est l'antigène S2.

8. Combinaison selon l'une quelconque des revendications 1 à 7, dans laquelle la combinaison est sous la forme d'un polypeptide de fusion.

9. Combinaison selon l'une quelconque des revendications 1 à 8, dans laquelle la combinaison est sous la forme dudit premier antigène de HCV et desdits antigènes supplémentaires liés à une matrice solide commune.

10. Combinaison selon la revendication 9, dans laquelle la matrice solide est la surface d'un puits de plaque de microtitration, une bille ou une bandelette.

11. Combinaison selon l'une quelconque des revendications 1 à 7, dans laquelle la combinaison est immobilisée sur la surface d'une matrice solide adaptée pour la détection du HCV par un dosage immunologique.

12. Procédé de détection d'anticorps dirigés contre HCV dans un constituant corporel de mammifère suspecté de contenir lesdits anticorps, comprenant la mise en contact dudit constituant corporel avec une combinaison d'antigènes selon l'une quelconque des revendications 1 à 11 dans des conditions qui permettent la réaction antigène anticorps et la détection de la présence de complexes immuns desdits anticorps et desdits antigènes.

13. Kit pour réaliser un dosage de détection d'anticorps dirigés contre un antigène de l'hépatite C (HCV) dans un constituant corporel de mammifère suspecté de contenir lesdits anticorps comprenant dans une combinaison conditionnée :
(a) la combinaison selon l'une quelconque des revendications 1 à 11 ;
(b) les réactifs témoins standard ; et
(c) les instructions pour réaliser le dosage.

## Revendications (Revendications pour l'(les) Etat(s) contractant(s) suivant(s): ES)

1. Procédé de détection d'anticorps dirigés contre le virus de l'hépatite C (HCV) dans un constituant corporel de mammifère suspecté de contenir lesdits anticorps, comprenant la mise en contact dudit constituant corporel avec une combinaison d'antigènes de HCV dans des conditions qui permettent la réaction anticorps-antigène et la détection de la présence de complexes immuns desdits anticorps et desdits antigènes, dans lequel les antigènes sont en un ou plusieurs polypeptides obtenus par synthèse chimique ou expression recombinante, immobilisés sur la surface d'une matrice solide adaptée pour la détection du HCV par un dosage immunologique, comprenant :
(a) un premier antigène de HCV comprenant un épitope du domaine C de la polyprotéine de HCV 1 représentée dans sur la figure 1 ; et
(b) un deuxième antigène de HCV choisi parmi
(i) un antigène de HCV comprenant un épitope du domaine S de la polyprotéine de HCV 1 représentée dans la figure 1 ;
(ii) un antigène de HCV comprenant un épitope du domaine NS3 de la polyprotéine de HCV 1 représentée dans la figure 1 ;
(iii) un antigène de HCV comprenant un épitope du domaine NS4 de la polyprotéine de HCV 1 représentée dans la figure 1 ; et
(iv) un antigène de HCV comprenant un épitope du domaine NS5 de la polyprotéine de HCV 1 représentée dans la figure 1;
sous réserve que la combinaison ne soit pas le peptide p1 avec C100-3, le peptide p35 avec C100-3, le peptide p99 avec C100-3 ou un peptide ayant les acides aminés 9 à 177 de la polyprotéine de HCV 1.

2. Procédé selon la revendication 1, dans lequel ledit deuxième antigène comprend un épitope du domaine S de la polyprotéine de HCV 1 représentée dans la figure 1, et laquelle combinaison comprend en outre au moins un antigène de HCV supplémentaire choisi parmi
(i) un antigène de HCV comprenant un épitope du domaine NS3 de la polyprotéine de HCV 1 représentée dans la figure 1 ;
(ii) un antigène de HCV comprenant un épitope du domaine NS4 de la polyprotéine de HCV 1 représentée dans la figure 1 ; et
(iii) un antigène de HCV comprenant un épitope du domaine NS5 de la polyprotéine de HCV 1 représentée dans la figure 1.

3. Procédé selon la revendication 2, dans lequel l'antigène supplémentaire provient du domaine NS3 de la polyprotéine de HCV 1 représentée dans la figure 1.

4. Procédé selon la revendication 2, dans lequel le premier antigène de HCV est C22 et l'antigène de HCV supplémentaire est C33c.

5. Procédé selon la revendication 2, dans lequel l'antigène supplémentaire provient du domaine NS4 de la polyprotéine de HCV 1 représentée dans la figure 1.

6. Procédé selon la revendication 5, dans lequel le premier antigène est C22 et le deuxième antigène de HCV est C100.

7. Procédé selon l'une quelconque des revendications 1 ou 6, dans lequel l'antigène du domaine S de la polyprotéine de HCV 1 représentée dans la figure 1 est l'antigène S2.

8. Procédé selon l'une quelconque des revendications 1 à 7, dans lequel la combinaison est sous la forme d'un polypeptide de fusion.

9. Procédé selon l'une quelconque de revendications 1 à 8, dans lequel la combinaison est sous la forme dudit premier antigène de HCV et desdits antigènes supplémentaires liés à une matrice solide commune.

10. Procédé selon la revendication 8, dans lequel la matrice solide est la surface d'un puits de plaque de microtitration, une bille ou une bandelette.

11. Combinaison d'antigène du virus de l'hépatite C (HCV) en un ou plusieurs polypeptides obtenus par synthèse chimique ou expression recombinante, comprenant :
(a) un premier antigène de HCV comprenant un épitope du domaine C de la polyprotéine de HCV 1 représentée dans la figure 1 ; et
(b) un deuxième antigène de HCV choisi parmi
(i) un antigène de HCV comprenant un épitope du domaine S de la polyprotéine de HCV 1 représentée dans la figure 1 ;
(ii) un antigène de HCV comprenant un épitope du domaine NS3 de la polyprotéine de HCV 1 représentée dans la figure 1 ;
(iii) un antigène de HCV comprenant un épitope du domaine NS4 de la polyprotéine de HCV 1 représentée dans la figure 1 ; et
(iv) un antigène de HCV comprenant un épitope du domaine NS5 de la polyprotéine de HCV 1 représentée dans la figure 1;
Sous réserve que la combinaison ne soit pas le peptide p1 avec C100-3, le peptide p35 avec C100-3, le peptide p99 avec C100-3 ou un peptide ayant les acides aminés 9 à 177 de la polyprotéine de HCV 1.

12. Combinaison selon la revendication 11, dans laquelle ledit deuxième antigène comprend un épitope du domaine S de la polyprotéine de HCV 1 représentée dans la figure 1, et laquelle combinaison comprend en outre au moins un antigène supplémentaire choisi parmi
(i) un antigène de HCV comprenant un épitope du domaine NS3 de la polyprotéine de HCV 1 représentée dans la figure 1 ;
(ii) un antigène de HCV comprenant un épitope du domaine NS4 de la polyprotéine de HCV 1 représentée dans la figure 1 ; et
(iii) un antigène de HCV comprenant un épitope du domaine NS5 de la polyprotéine de HCV 1 représentée dans la figure 1.

13. Combinaison selon la revendication 12, dans laquelle l'antigène supplémentaire provient du domaine NS3 de la polyprotéine de HCV 1 représentée dans la figure 1.

14. Combinaison selon la revendication 13, dans laquelle le premier antigène de HCV est C22 et l'antigène de HCV supplémentaire est C33c.

15. Combinaison selon la revendication 14, dans laquelle l'antigène supplémentaire provient du domaine NS4 de la polyprotéine de HCV 1 représentée dans la figure 1.

16. Combinaison selon une quelconque revendication 15, dans laquelle le premier antigène est C22 et le deuxième antigène de HCV est C 100.

17. Combinaison selon l'une quelconque des revendications 11 à 16, dans laquelle l'antigène du domaine S de la polyprotéine de HCV 1 représentée dans la figure 1 est l'antigène S2.

18. Combinaison selon l'une quelconque des revendications 11 à 17, dans laquelle la combinaison est sous la forme d'un polypeptide de fusion.

19. Combinaison selon l'une quelconque des revendications 11 à 18, dans laquelle la combinaison est sous la forme dudit premier antigène de HCV et desdits antigènes supplémentaires liés à une matrice solide commune.

20. Combinaison selon la revendication 19, dans laquelle la matrice solide est la surface d'un puits de plaque de microtitration, une bille ou une bandelette.

21. Combinaison selon l'une quelconque des revendications 11 à 20, dans laquelle la combinaison est immobilisée sur la surface d'une matrice solide adaptée pour la détection du HCV par un test immunologique.

22. Kit pour réaliser un dosage afin de détecter des anticorps contre un antigène de l'hépatite C (HCV) dans un constituant corporel de mammifère suspecté de contenir lesdits anticorps comprenant en combinaison conditionnée :
(a) la combinaison selon l'une quelconque des revendications 11 à 21 ;
(b) les réactifs témoins standard ; et
(c) les instructions pour réaliser le test.
